(19) Europäisches Patentamt / European Patent Office / Office européen des brevets

(11) **EP 4 736 739 A1**

(12) **EUROPEAN PATENT APPLICATION**
published in accordance with Art. 153(4) EPC

(43) Date of publication:
06.05.2026 Bulletin 2026/19

(21) Application number: 24831691.1

(22) Date of filing: 13.06.2024

(51) International Patent Classification (IPC):
*A61B 3/10* (2006.01)    *A61B 3/12* (2006.01)

(52) Cooperative Patent Classification (CPC):
A61B 3/10; A61B 3/12

(86) International application number:
PCT/JP2024/021492

(87) International publication number:
WO 2025/004826 (02.01.2025 Gazette 2025/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 27.06.2023 JP 2023105356

(71) Applicant: Topcon Corporation
Tokyo 174-8580 (JP)

(72) Inventors:
• MINO Toshihiro
Tokyo 174-8580 (JP)
• MORIGUCHI Yoshikiyo
Tokyo 174-8580 (JP)
• ISHIKAWA Akiko
Tokyo 174-8580 (JP)

(74) Representative: Vossius & Partner
Patentanwälte Rechtsanwälte mbB
Siebertstraße 3
81675 München (DE)

(54) **ANALYSIS PROCESSING DEVICE, OPTICAL COHERENCE TOMOGRAPHY DEVICE, ANALYSIS PROCESSING METHOD, AND PROGRAM**

(57) This analysis processing device includes an acquisition unit, an analysis unit, and a display control unit. The acquisition unit acquires two or more items of motion contrast data for the same cross-section of an object under measurement at mutually different inter-scan time intervals, the motion contrast data being obtained by repeating a B-scan three or more times on the cross-section. The analysis unit executes an analysis process for deriving the temporal variation of motion contrast intensity in the cross-section on the basis of the two or more items of motion contrast data. The display control unit causes a display means to display an image representing the temporal variation obtained using the analysis process.

FIG. 2

ANALYSIS PROCESSING APPARATUS — 1100
ACQUISITION UNIT — 1110
ANALYZER — 1120
VISTA PROCESSOR — 1121
FLOW PARAMETER ANALYZER — 1122
DISPLAY CONTROLLER — 1130

**Description**

[TECHNICAL FIELD]

The present invention relates to an analysis processing apparatus, an optical coherence tomography apparatus, an analysis processing method, and a program.

[BACKGROUND ART]

Optical coherence tomography (OCT) that is used for forming images representing the surface morphology or the internal morphology of an object to be measured using light beam emitted from a laser light source or the like have been known. OCT is not invasive on the human body, and therefore is expected to be applied to the medical field or the biological field, in particular. For example, in the ophthalmic field, apparatuses for forming images of the fundus, the cornea, or the like have been in practical use. Such apparatuses using a method of OCT (OCT apparatuses) can be applied to observe various sites of an eye to be examined. In addition, because of the ability to acquire high-definition images, the OCT apparatuses are applied to the diagnosis of various eye diseases.

In particular, OCT angiography (OCTA) has recently attracted attention as a method for imaging components that change within the minute time between OCT scans by repeatedly performing OCT scans on the same location of the object to be measured. Such OCTA is used as a method for visualizing information on a structure of a vasculature in a retina and/or a choroid within a living body.

For example, Patent Document 1, Patent Document 2, and Non-Patent Document 1 disclose Variable Interscan Time Analysis (VISTA) for analyzing a relative blood flow velocity by utilizing a dependency of OCT signals (OCTA images) in the interscan time (time between scans).

For example, as described in Non-Patent Document 1, a relative blood flow distribution in the retinal/choroidal vessel layer such as a superficial layer of the retina, a deep layer of the retina, and a choriocapillaris can be provided by using VISTA. VISTA is expected to be used to evaluate the activity of blood vessels, especially capillary or neovascular.

[PRIOR ART DOCUMENTS]

[PATENT DOCUMENTS]

[PATENT DOCUMENT 1] U.S. Unexamined Patent Application Publication No. 2018/0315194
[PATENT DOCUMENT 2] U.S. Unexamined Patent Application Publication No. 2020/0064118

[NON-PATENT DOCUMENTS]

[NON-PATENT DOCUMENT 1] Stefan B. Ploner et al., "Toward quantitative OCT angiography: visualizing blood flow speeds in ocular pathology using variable interscan time analysis (VISTA)", Retina. Auther manuscript; available in PMC 2017 December 01

[SUMMARY OF THE INVENTION]

[PROBLEMS TO BE SOLVED BY THE INVENTION]

Generally, three-dimensional volume data of the object to be measured is acquired by performing OCT scan, the region of interest is identified in a projection image generated from the acquired volume data, and VISTA is performed to observe the identified region of interest in detail. In such conventional VISTA, it is practically impossible to acquire results of time analysis, such as time variation of relative blood flow velocity, in real time.

Further, VISTA requires repeatedly performing OCT scans on the same location of the object to be measured. Thus, compared to OCTA, VISTA significantly increases both measurement time and analysis time, while also requiring a significantly larger amount of data for analysis. As a result, conventional VISTA cannot acquire analysis results in real time, and while VISTA can provide spatial distribution of relative blood flow velocity, VISTA cannot provide the time variation of relative blood flow velocity.

Furthermore, in VISTA, it is necessary to adjust the scan parameters of the OCT scan according to the region of interest in order to acquire useful time analysis results. However, VISTA has no means to adjust the scan parameters while observing the region of interest in the object to be measured in real time.

The present invention has been made in view of such circumstances, and one of objects of the present invention is to provide a new technique for acquiring time analysis result of VISTA for the object to be measured in real time.

[MEANS OF SOLVING THE PROBLEMS]

One aspect of embodiments is an analysis processing apparatus including: an acquisition unit configured to acquire two or more pieces of motion contrast data of a cross-section at interscan time intervals different from each other, the two or more pieces of motion contrast data being acquired by repeatedly performing B-scans three or more times on the same cross-section in an object to be measured; an analyzer configured to perform analysis processing for obtaining a time variation of a motion contrast intensity in the cross-section based on the two or more pieces of motion contrast data; and a display controller configured to cause an image representing the time variation obtained by performing the analysis processing to be displayed on a display means.

Another aspect of the embodiments is an optical coherence tomography apparatus including: an optical system configured to perform optical coherence tomography on the object to be measured; and the analysis processing apparatus described above, the analysis processing apparatus being configured to cause an image representing the time variation to be displayed on the display means based on the two or more pieces of motion contrast data acquired by repeatedly performing three or more B-scans on the same cross-section in the object to be measured using the optical system.

Still another aspect of the embodiments is an analysis processing method including: an acquisition step of acquiring two or more pieces of motion contrast data of a cross-section at interscan time intervals different from each other, the two or more pieces of motion contrast data being acquired by repeatedly performing B-scans three or more times on the same cross-section in an object to be measured; an analyzing step of performing analysis processing for obtaining a time variation of a motion contrast intensity in the cross-section based on the two or more pieces of motion contrast data; and a display control step of causing an image representing the time variation obtained by performing the analysis processing to be displayed on a display means.

Still another aspect of the embodiments is a program of causing a computer to execute each step of the analysis processing method described above.

Still another aspect of the embodiments is a computer program product including the computer program(s)/instruction(s). The computer program product realizes each step in the analysis processing method described above, when the computer program(s)/instruction(s) is/are executed by the processor.

Still another aspect of the embodiments is a computer readable storage medium (recording medium) in which the computer program(s)/instruction(s) is/are stored. The computer readable storage medium realizes each step in the analysis processing method according to the embodiments, when the computer program(s)/instruction(s) is/are executed by the processor.

[EFFECTS OF THE INVENTION]

According to the present invention, a new technique for acquiring time analysis result of VISTA for the object to be measured in real time can be provided.

[BRIEF EXPLANATION OF THE DRAWINGS]

[FIG. 1] FIG. 1 is a schematic diagram illustrating an example of a configuration of an analysis system according to embodiments.

[FIG. 2] FIG. 2 is a schematic diagram illustrating an example of a configuration of an analysis processing apparatus according to the embodiments.

[FIG. 3] FIG. 3 is a schematic diagram for explaining a VISTA according to the embodiments.

[FIG. 4] FIG. 4 is a schematic diagram for explaining the VISTA according to the embodiments.

[FIG. 5] FIG. 5 is a schematic diagram illustrating an example of a configuration of a VISTA processor according to the embodiments.

[FIG. 6] FIG. 6 is a schematic diagram for explaining the VISTA according to the embodiments.

[FIG. 7] FIG. 7 is a schematic diagram for explaining the VISTA according to the embodiments.

[FIG. 8] FIG. 8 is a schematic diagram for explaining the VISTA according to the embodiments.

[FIG. 9] FIG. 9 is a schematic diagram illustrating another example of a configuration of the analysis system according to the embodiments.

[FIG. 10] FIG. 10 is a schematic diagram illustrating an example of a configuration of an optical system of an ophthalmic apparatus according to the embodiments.

[FIG. 11] FIG. 11 is a schematic diagram illustrating an example of the configuration of the optical system of the ophthalmic apparatus according to the embodiments.

[FIG. 12] FIG. 12 is a schematic diagram illustrating an example of the configuration of a processing system of the ophthalmic apparatus according to the embodiments.

[FIG. 13] FIG. 13 is a schematic diagram illustrating an example of the configuration of the processing system of the ophthalmic apparatus according to the embodiments.

[FIG. 14] FIG. 14 is a schematic diagram illustrating an example of the configuration of the processing system of the ophthalmic apparatus according to the embodiments.

[FIG. 15] FIG. 15 is a flowchart of an example of an operation of the ophthalmic apparatus according to the embodiments.

[FIG. 16] FIG. 16 is a flowchart of an example of the operation of the ophthalmic apparatus according to the embodiments.

[FIG. 17] FIG. 17 is a flowchart of an example of the operation of the ophthalmic apparatus according to the embodiments.

[FIG. 18] FIG. 18 is a schematic diagram for explaining the operation of the ophthalmic apparatus according to the embodiments.

[FIG. 19] FIG. 19 is a schematic diagram for explaining the operation of the ophthalmic apparatus according to the embodiments.

[FIG. 20] FIG. 20 is a flowchart of an example of the operation of the ophthalmic apparatus according to the embodiments.

[FIG. 21] FIG. 21 is a flowchart of an example of the operation of the ophthalmic apparatus according to the embodiments.

[MODES FOR CARRYING OUT THE INVENTION]

Referring now to the drawings, exemplary embodiments of an analysis processing apparatus, an optical coherence tomography apparatus, an analysis processing method, and a program according to the present invention are described below. In the embodiments, any of the techniques disclosed in the documents cited in the present specification can be applied to the embodiments below.

An analysis processing apparatus according to embodiments is configured to acquire two or more pieces of motion contrast data of a cross-section at interscan time intervals different from each other. Here, the two or more pieces of motion contrast data are acquired by repeatedly performing B-scans three or more times on the (approximately) same cross-section in an object to be measured. The analysis processing apparatus is configured to perform analysis processing for obtaining a time variation of a motion contrast intensity in the cross-section based on the acquired two or more pieces of motion contrast data, and to cause an image representing the time variation of the motion contrast intensity, that is obtained by performing the analysis processing, to be displayed on a display means.

The motion contrast data is data representing a distribution of the contrast intensity that varies due to motion. The motion contrast data is generated from a difference (or decorrelation components) of two pieces of B-scan data acquired by performing B-scans on the same cross-section in the object to be measured at scan timings different from each other. The interscan time corresponds to the time between two B-scan timings. Examples of the motion contrast data include a motion contrast image.

Examples of the image representing the time variation of the motion contrast intensity include an image in which luminance information corresponding to statistical value of the motion contrast intensity and hue information corresponding to a saturation time of the motion contrast intensity are assigned for each pixel.

This makes it possible to perform B-scan image based VISTA (variable interscan time analysis). Thus, the measurement time and the time required for analysis can be significantly reduced, and the amount of data required for analysis can also be significantly decreased. As a result, it is possible to generate VISTA analysis results in real time, and it is also possible to cause the VISTA analysis results to be displayed on the display means in real time.

In addition, since the VISTA analysis results can be generated in real time, scan parameters including interscan time and the number of B-scan repetitions can be easily optimized, which also improves the accuracy of the analysis results.

Furthermore, it is possible to automatically or manually designate a vascular region in the B-scan image, to perform time analysis of relative blood flow in the designated vascular region, and to acquire the time analysis results of relative blood flow in real time.

In some embodiments, the analysis processing apparatus is configured to generate the two or more pieces of motion contrast data of the cross-section in the object to be measured at interscan time intervals different from each other, from measurement data acquired by an external measurement apparatus. In some embodiments, the analysis processing apparatus is configured to repeatedly perform B-scan three or more times on the same cross-section in the object to be measured, and to generate two or more pieces of motion contrast data of the cross-section in the object to be measured at interscan time intervals different from each other from the acquired measurement data.

The B-scan according to the embodiments may be a B-scan using ultrasound or a B-scan using electromagnetic waves including light. Examples of the B-scan using light include B-scan using optical coherence tomography (OCT).

An analysis processing method according to the embodiments includes one or more steps performed by the analysis processing apparatus described above. A program (computer program)/instruction according to the embodiments causes a computer (processor) to execute each step in the analysis processing method according to the embodiments. A computer program product according to the embodiments includes the computer program(s)/instruction(s). The computer program product realizes each step in the analysis processing method according to the embodiments, when the computer program(s)/instruction(s) is executed by the processor. A recording medium according to the embodiments is a computer readable non-transitory recording medium (storage medium) on which the program according to the embodiments is recorded (stored). The computer readable recording medium according to the embodiments stores the computer program(s)/instruction(s). The computer readable recording medium realizes each step in the analysis processing method according to the embodiments, when the computer program(s)/instruction(s) is executed by the processor.

In this specification, the processor includes, for example, a circuit(s) such as, for example, a CPU (central processing unit), a GPU (graphics processing unit), an ASIC (application specific integrated circuit), and a PLD (programmable logic device). Examples of PLD include a simple programmable logic device (SPLD), a complex programmable logic device (CPLD), and a field programmable gate array (FPGA). The processor realizes, for example, the function according to the embodiments by reading out a computer program stored in a storage circuit or a storage device and executing the computer program. At least a part of the storage circuit or the storage apparatus may be included in the processor. Further, at least a part of the storage circuit or the storage apparatus may be provided outside of the processor.

Hereinafter, a case where VISTA processing is performed using motion contrast data acquired by performing OCT B-scan on a living eye as the object to be measured will be mainly described. However, the embodiments described below can be applied to a case where B-scan other than OCT is performed on the object to be measured. Further, the embodiments described below can be applied to a case where OCT B-scan is performed on the object to measured other than the living eye. Furthermore, the embodiments described below can be applied to a case where B-scan other than OCT is performed on the object to be measured other than the living eye.

< Analysis system >

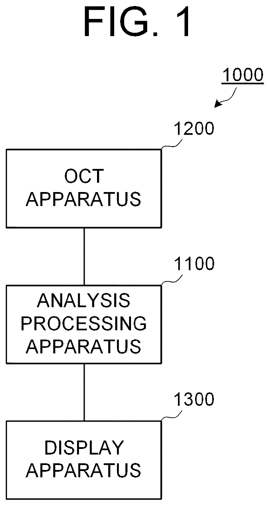

FIG. 1 shows a block diagram of an example of a configuration of an analysis system according to the embodiments.

The analysis system 1000 includes an analysis processing apparatus 1100, an OCT apparatus 1200, and a display apparatus 1300.

The OCT apparatus 1200 includes an OCT optical system. The OCT optical system includes an interference optical system. The interference optical system is configured to split light from an light source into measurement light and reference light, to irradiate the object to be measured with the split measurement light, to make returning light of the measurement light from the object to be measured and the reference light having traveled through a reference optical path interfere with each other to generate interference light, and to detect the interference light. The OCT apparatus 1200 generates OCT data (OCT image) based on the detected interference light, and generates OCTA data (OCTA image) from formed two or more pieces of OCT data.

Specifically, the OCT apparatus 1200 repeatedly performs OCT B-scans three or more times on the same cross-section in the object to be measured, and sequentially forms three or more OCT images (B-scan images, tomographic images) representing the structure of the cross-section based on the obtained measurement data. Further, the OCT apparatus 1200 generates the two or more pieces of motion contrast data of the cross-section at interscan time intervals different from each other, from the formed three or more OCT images. Example of the motion contrast data includes an OCTA image and OCTA data.

The analysis processing apparatus 1100 acquires the two or more pieces of motion contrast data generated by the OCT apparatus 1200, and performs analysis processing for obtaining a time variation of a motion contrast intensity in the cross-section based on the acquired two or more pieces of motion contrast data. This analysis processing may be the processing for performing VISTA. The analysis processing apparatus 1100 causes an image representing the time variation of the motion contrast intensity obtained by performing the analysis processing to be displayed on the display apparatus 1300, in real time.

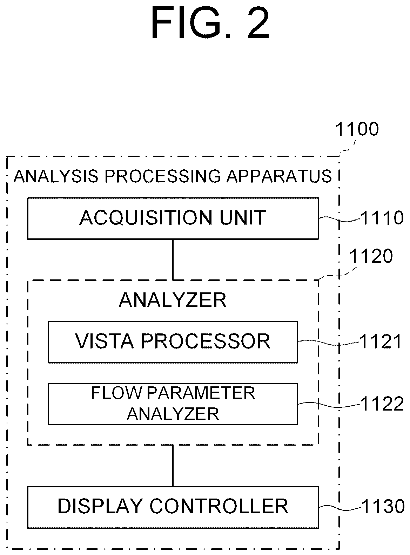

FIG. 2 shows a block diagram of an example of the configuration of the analysis processing apparatus 1100 in FIG. 1. In FIG. 2, like reference numerals designate like parts as in FIG. 1. The same description may not be repeated.

The analysis processing apparatus 1100 includes an acquisition unit 1110, an analyzer 1120, and a display controller 1130. The acquisition unit 1110 acquires the two or more pieces of motion contrast data of the cross-section at interscan time intervals different from each other. Here, the two or more pieces of motion contrast data are generated by the OCT apparatus 1200.

For example, the function of the acquisition unit 1110 is realized by a communication unit that establishes a communication connection with the OCT apparatus 1200. In some embodiments, the acquisition unit 1110 is configured to acquire three or more OCT images (OCT data) sequentially formed by the OCT apparatus 1200, and to generate the two or more pieces of motion contrast data of the cross-section at interscan time intervals different from each other from the acquired three or more OCT images.

The analyzer 1120 includes a VISTA processor 1121 and a flow parameter analyzer 1122.

The VISTA processor 1121 identifies the time variation of motion contrast intensity in the cross-section, by performing VISTA processing using the two or more pieces of motion contrast data acquired by the acquisition unit 1110. The VISTA processor 1121 generates a VISTA image representing a distribution of the time variability of the identified motion contrast intensity as an analysis result.

The flow parameter analyzer 1122 identifies the time variation of the motion contrast intensity at a designated position in the VISTA image generated by the VISTA processor 1121 as a flow parameter. The position designated in the VISTA image may be a position designated by a user, or a position designated by analyzing the VISTA image.

The flow parameter analyzer 1122 can generate information representing a time change of the time variation of the motion contrast intensity at a designated position in the cross-section. For example, the acquisition unit 1110 acquires two or more contrast data groups acquired at timings different from each other. Here, each of the two or more contrast data groups includes the two or more pieces of motion contrast data described above. In this case, the VISTA processor 1121 sequentially performs VISTA on each of the two or more contrast data groups, and sequentially generates two or more VISTA images corresponding to each of the two or more contrast data groups. The flow parameter analyzer 1122 identifies generated the time variation of the motion contrast intensity at the designated position in each of the two or more VISTA images as a flow parameter, and generates information representing a time change in the flow parameter.

The display controller 1130 causes the analysis results obtained by performing analysis processing executed by the analyzer 1120 to be displayed on the display apparatus 1300, in real time. For example, the display controller 1130 causes at least one of the VISTA image generated by the VISTA processor 1121, the information representing the time variation (flow parameter) of the motion contrast intensity generated by the flow parameter analyzer 1122, or the information representing the time change in the flow parameter to be displayed on the display apparatus 1300.

In addition, in case that the two or more contrast data groups described above are acquired by the acquisition unit 1110, the display controller 1130 can cause the two or more VISTA images sequentially generated by the VISTA processor 1121 to be simultaneously displayed on the screen of the display apparatus 1300. The display controller 1130 may cause the two or more VISTA images to be sequentially displayed (slow display, moving image display) in chronological order at the same position on the screen of display apparatus 1300.

It should be noted that the functions of the analysis processing apparatus 1100 shown in FIG. 2 are realized by one or more processors. In this case, the processor executes VISTA and control over the display apparatus 1300 (in some cases, furthermore, the generation of OCTA images and motion contrast data) according to a program stored in advance.

The following will describe VISTA according to the embodiments. Hereinafter, a living eye will be used as an example of the object to be measured.

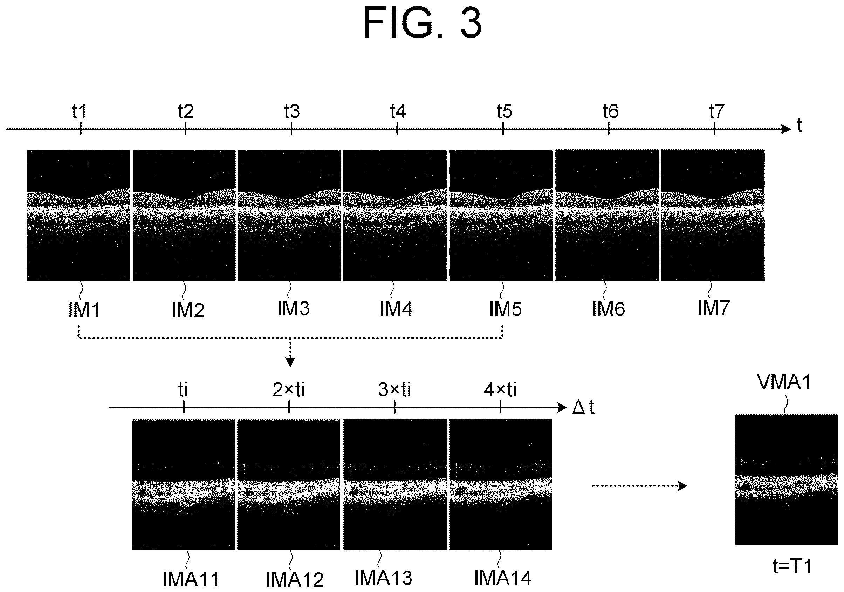

FIG. 3 to FIG. 8 show diagrams for explaining VISTA performed by the VISTA processor 1121 shown in FIG. 2.

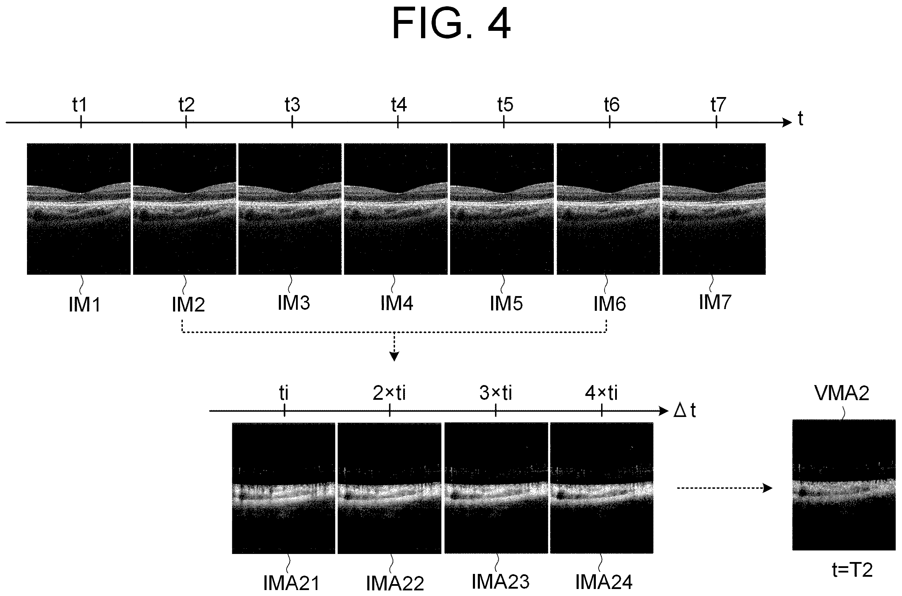

FIG. 3 schematically represents an example of the VISTA image at time t = T1. Here, the VISTA image is generated from five OCT images acquired by repeatedly performing OCT B-scans on a cross-section passing through the macula of the living eye. FIG. 4 schematically represents an example of the VISTA image at time t = T2 (T1 < T2). Here, the VISTA image is generated from five OCT images acquired by repeatedly performing OCT B-scans on the same cross-section as in FIG. 3. In FIG. 4, the same parts as in FIG. 3 are shown by the same symbols and their descriptions will be omitted in an appropriate manner.

The OCT apparatus 1200 repeatedly performs OCT B-scans on the same cross-section in the macula of the living eye. For example, as shown in FIG. 3, the OCT apparatus 1200 performs B-scans at times t1, t2, ..., t7, and sequentially forms B-scan images IM1 to IM7 at each B-scan scan timing. Hereafter, the time interval (interscan time interval) from time t1 to t7 is assumed to be the same. However, the time intervals from time t1 to t7 may differ from each other.

Subsequently, the OCT apparatus 1200 (or analysis processing apparatus 1100) generates four OCTA images IMA11 to IMA14 at interscan time intervals different from each other, from the B-scan images IM1 to IM5 formed first among the B-scan images IM1 to IM7, as shown in FIG. 3. The image sizes of the four OCTA images IMA11 to IMA14 are identical, and each position of the living eye is depicted at the same pixel position in each of the OCTA images IMA11 to IMA14.

The OCTA image IMA11 is generated from the difference of two B-scan images with an interscan time interval $\Delta t$ = ti, which is the scan timing interval. The two B-scan images are the B-scan images IM1 and IM2, the B-scan images IM2 and IM3, the B-scan images IM3 and IM4, or the B-scan images IM4 and IM5.

In some embodiments, the OCTA image IMA11 is an averaged image of two or more of OCTA images among the following OCTA images: an OCTA image generated from the difference between the B-scan images IM1 and IM2; an OCTA image generated from the difference between the B-scan images IM2 and IM3; an OCTA image generated from the difference between the B-scan images IM3 and IM4; and an OCTA image generated from the difference between the B-scan images IM4 and IM5.

The OCTA image IMA12 is generated from the difference of two B-scan images with an interscan time interval $\Delta t =$ "2 x ti", which is the scan timing interval. The two B-scan images are the B-scan images IM1 and IM3, the B-scan images IM2 and IM4, or the B-scan images IM3 and IM5.

In some embodiments, the OCTA image IMA12 is an averaged image of two or more of OCTA images among the following OCTA images: an OCTA image generated from the difference between the B-scan images IM1 and IM3; an OCTA image generated from the difference between the B-scan images IM2 and IM4; and an OCTA image generated from the difference between the B-scan images IM3 and IM5.

The OCTA image IMA13 is generated from the difference of two B-scan images with an interscan time interval $\Delta t =$ "3 x ti", which is the scan timing interval. The two B-scan images are the B-scan images IM1 and IM4, or the B-scan images IM2 and IM5.

In some embodiments, the OCTA image IMA13 is an averaged image of the following two OCTA image: an OCTA image generated from the difference between the B-scan images IM1 and IM4; and an OCTA image generated from the difference between the B-scan images IM2 and IM5.

The OCTA image IMA14 is generated from the difference of two B-scan images with an interscan time interval $\Delta t =$ "4 x ti", which is the scan timing interval. The two B-scan images are the B-scan images IM1 and IM5.

The analysis processing apparatus 1100 acquires the OCTA images IMA11 to IMA14 described above, which are sequentially generated by the OCT apparatus 1200, and generates the VISTA image VMA1 at time t = T1 by analyzing the acquired OCTA images IMA11 to IMA14. Time t = T1 may be a time of scan timing of any of the OCTA images IMA11 to IMA14 that were used to generate the VISTA image VMA1, or a statistical value (mean or median) of the scan timing (time) of the OCTA images IMA11 to IMA14 that were used to generate the VISTA image VMA1.

In the same way, the OCT apparatus 1200 (or analysis processing apparatus 1100) generates four OCTA images IMA21 to IMA24 at interscan time intervals different from each other, from the B-scan images IM2 to IM6 among the B-scan images IM1 to IM7, as shown in FIG. 4.

The OCTA image IMA21 is generated from the difference of two B-scan images with an interscan time interval $\Delta t =$ ti, which is the scan timing interval. The two B-scan images are the B-scan images IM2 and IM3, the B-scan images IM3 and IM4, the B-scan images IM4 and IM5, or the B-scan images IM5 and IM6.

In some embodiments, the OCTA image IMA21 is an averaged image of two or more of OCTA images among the following OCTA images: an OCTA image generated from the difference between the B-scan images IM2 and IM3; an OCTA image generated from the difference between the B-scan images IM3 and IM4; an OCTA image generated from the difference between the B-scan images IM4 and IM5; and an OCTA image generated from the difference between the B-scan images IM5 and IM6.

The OCTA image IMA22 is generated from the difference of two B-scan images with an interscan time interval $\Delta t =$ "2 x ti", which is the scan timing interval. The two B-scan images are the B-scan images IM2 and IM4, the B-scan images IM3 and IM5, or the B-scan images IM4 and IM6.

In some embodiments, the OCTA image IMA22 is an averaged image of two or more of OCTA images among the following OCTA images: an OCTA image generated from the difference between the B-scan images IM2 and IM4; an OCTA image generated from the difference between the B-scan images IM3 and IM5; and an OCTA image generated from the difference between the B-scan images IM4 and IM6.

The OCTA image IMA23 is generated from the difference of two B-scan images with an interscan time interval $\Delta t =$ "3 x ti", which is the scan timing interval. The two B-scan images are the B-scan images IM2 and IM5, or the B-scan images IM3 and IM6.

In some embodiments, the OCTA image IMA23 is an averaged image of the following two OCTA image: an OCTA image generated from the difference between the B-scan images IM2 and IM5; and an OCTA image generated from the difference between the B-scan images IM3 and IM6.

The OCTA image IMA24 is generated from the difference of two B-scan images with an interscan time interval $\Delta t =$ "4 x ti", which is the scan timing interval. The two B-scan images are the B-scan images IM2 and IM6.

The analysis processing apparatus 1100 acquires the OCTA images IMA21 to IMA24 described above, which are sequentially generated by the OCT apparatus 1200, and generates the VISTA image VMA2 at time t = T2 by analyzing the acquired OCTA images IMA21 to IMA24. Time t = T2 may be a time of scan timing of any of the OCTA images IMA21 to IMA24 that were used to generate the VISTA image VMA2, or a statistical value (mean or median) of the scan timing (time) of the OCTA images IMA21 to IMA24 that were used to generate the VISTA image VMA2.

The OCT apparatus 1200 (or analysis processing apparatus 1100) can similarly generate four OCTA images (contrast data group) sequentially at interscan time intervals different from each other, and sequentially generate VISTA image from the four generated OCTA images.

In FIG. 3 and FIG. 4, the time interval of time t1 to t7 need not be identical. In this case, it is possible to acquire a plurality of OCTA images with unequal interscan time intervals from a plurality of OCT images at time t1 to t7, and to analyze the time variation of motion contrast intensity in accordance with the unequal interscan time interval of each of the acquired OCTA images.

The VISTA processor 1121 in FIG. 2 generates the VISTA images from the four OCTA images.

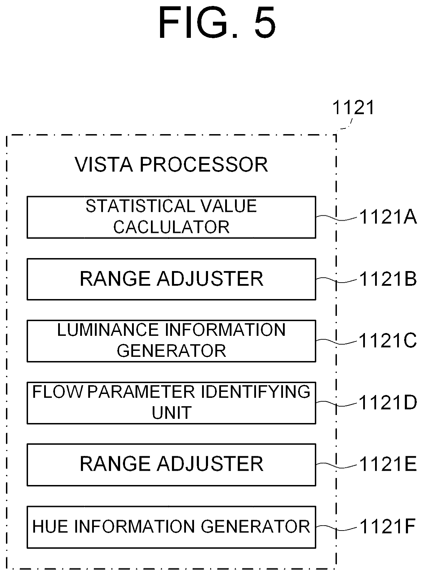

FIG. 5 represents a block diagram of an example of the configuration of the VISTA processor 1121 in FIG. 2.

The VISTA processor 1121 includes a statistical value calculator 1121A, a range adjuster 1121B, a luminance information generator 1121C, a flow parameter identifying unit 1121D, a range adjuster 1121E, and a hue information generator 1121F. The VISTA processor 1121 generates luminance information and hue information from the four OCTA images for each pixel, and generates a single VISTA image by assigning the generated luminance information and hue information to each pixel.

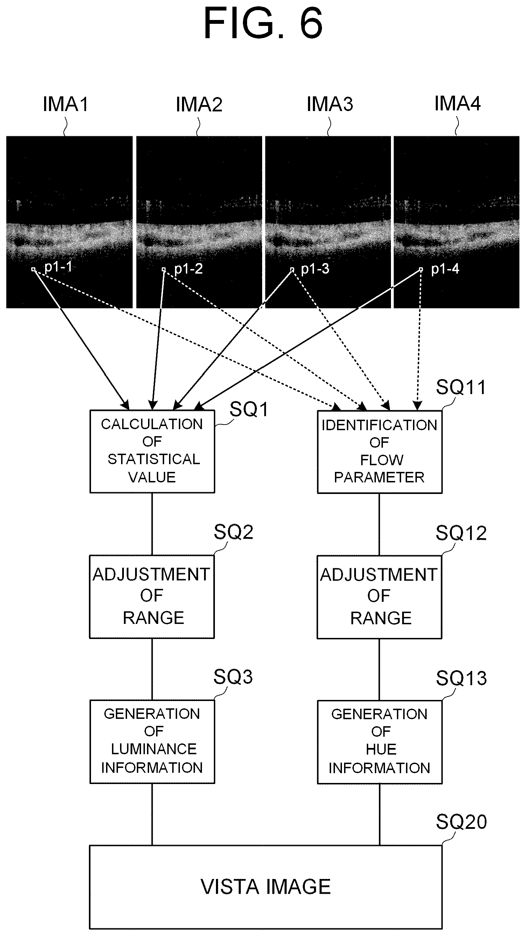

FIG. 6 represents an explanatory diagram of an operation of the VISTA processor 1121 in FIG. 5. In FIG. 6, the four OCTA images IMA1 to IMA4 correspond to the OCTA images IMA11 to IMA14 in FIG. 3, or the OCTA images IMA21 to IMA24 in FIG. 4. A pixel position (image region) p1-1 in the OCTA image IMA1, a pixel position pl-2 in the OCTA image IMA2, a pixel position p1-3 in the OCTA image IMA3, and a pixel position p1-4 in the OCTA image IMA4, respectively, indicate the same position of the living eye. It should be noted that, in FIG. 6, a case where the VISTA image is generated from the four OCTA images will be shown. However, a case where the VIST image is generated from two, three, or five or more OCTA images are also similar.

The statistical value calculator 1121A calculates a statistical value of pixel values at pixel positions p1-1 to p1-4 in the four OCTA images IMA1 to IMA4. Specifically, the statistical value calculator 1121A calculates the statistical values of the pixel values at the same pixel positions in the four OCTA images IMA1 to IMA4 for each pixel, for all pixel positions in the four OCTA images IMA1-IMA4 (SQ1 in FIG. 6). Examples of the statistical value include a maximum value, a minimum value, a median, an average value, a mode, a variance, a standard deviation, and the value of a predetermined evaluation equation using any of the above statistical values. In the present embodiment, the statistical value is assumed to be the maximum value.

The range adjuster 1121B adjusts a range of the luminance information so that all statistical values for all pixel positions, which are calculated in SQ1, fall within the range, and assigns all predetermined luminance values within the adjusted range (SQ2).

The luminance information generator 1121C generates the luminance information by assigning the statistical values calculated in SQ1 to the luminance values assigned in SQ2 for each of all pixel positions in the four OCTA images IMA1 to IMA4 (SQ3).

On the other hand, the flow parameter identifying unit 1121D identifies the flow parameter from the pixel values at the pixel positions p1-1 to pl-4 in the four OCTA images IMA1 to IMA4. Here, the flow parameter is a value corresponding to the time variation of the motion contrast intensity. The flow parameter identifying unit 1121D identifies the flow parameters from the pixel values at the same pixel positions in the four OCTA images IMA1 to IMA4 for each pixel, for all pixel positions in the four OCTA images IMAI-IMA4 (SQ11).

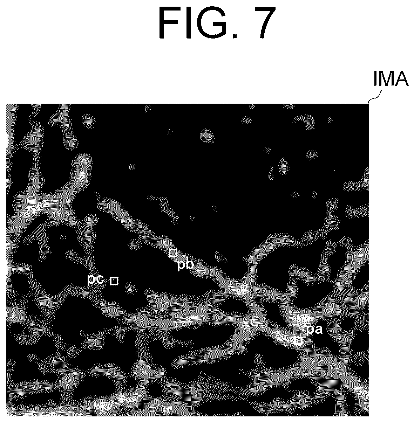

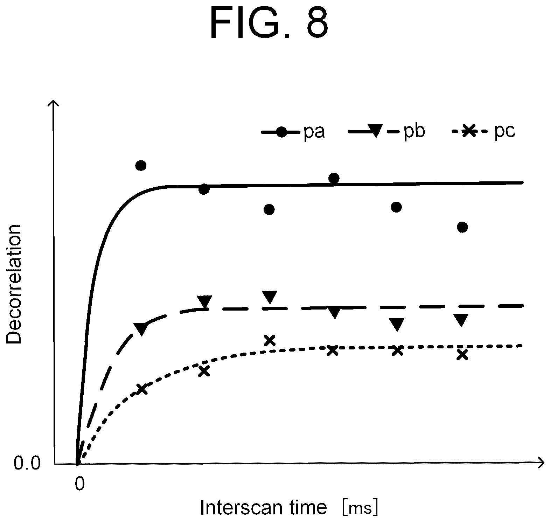

FIG. 7 and FIG. 8 show explanatory diagrams of an operation of the flow parameter identifying unit 1121D in FIG. 5. FIG. 7 represents an example of pixels (or image regions) pa, pb, and pc in the OCTA image IMA. The pixel pa is a pixel in a large vascular region. The pixel pc is a pixel in a small vascular region. The pixel pb is a pixel in a vascular region that is smaller than the vascular region where the pixel pa is positioned and is larger than the vascular region where the pixel pc is positioned. FIG. 8 schematically represents a saturation curves of the Decorrelation value (non-correlation value) at the pixels pa, pb, and pc. In FIG. 8, a vertical axis represents decorrelation value, while the horizontal axis represents interscan time. In FIG. 8, the flow parameters are assumed to be identified from six OCTA images.

The decorrelation value corresponds to the motion contrast, and is the value corresponding to the uncorrelated signal intensity in the OCTA image. The decorrelation value indicates that the more rapid the time variation of the decorrelation value, the faster the relative blood flow velocity becomes, and that the more gradual the time variation of the decorrelation value, the slower the relative blood flow velocity becomes. The flow parameter identifying unit 1121D performs fitting the decorrelation values of the corresponding pixels in the OCTA images to a first-order saturation function shown in Equation (1) for each of all pixel positions in the OCTA images. The flow parameter identifying unit 1121D obtains the time constant $\tau$ of the first-order saturation function as a saturation time from the first-order saturation function obtained by performing fitting, and identifies the inverse of the saturation time ($1 / \tau$) as the flow parameter (SQ11).

[Equation 1]

$$ Decorrelation(t) = D\left\{1 - exp\left(-\frac{t}{\tau}\right)\right\} \qquad (1) $$

In Equation (1), "t" is time and "D" is the saturation level of the decorrelation value.

The range adjuster 1121E adjusts a range of the hue information so that all flow parameters for all pixel positions, which are identifies in SQ11, fall within the range, and assigns all predetermined hue values within the adjusted range (SQ12).

The hue information generator 1121F generates the hue information by assigning the flow parameters calculated in SQ11 to the hue values assigned in SQ12 for each of all pixel positions in the four OCTA images IMA1 to IMA4 (SQ13). The hue information is information for expressing in hue that the larger the flow parameter, the faster the relative blood flow velocity becomes, and that the smaller the flow parameter, the slower the relative blood flow velocity becomes. For example, with reference to a predetermined reference relative blood flow velocity, hue values with faster relative blood flow velocity are assigned to warm colors, while hue values with slower relative blood flow velocity are assigned to cool colors.

In some embodiments, the hue information generator 1121F identifies the distribution of all flow parameters identified in SQ11 and identifies the mean, the median, or the mode of the identified distribution as the reference relative blood flow velocity. The hue information generator 1121F generates the hue information by assigning the calculated flow parameters to the hue values with reference to the identified reference relative blood flow velocity.

In some embodiments, the hue information generator 1121F generates the hue information by assigning the calculated flow parameters to the hue values with a predetermined fixed value as the reference relative blood flow velocity.

The VISTA processor 1121 generates a single VISTA image by assigning the luminance information generated in SQ3 and the hue information generated in SQ13 to the pixel for each pixel (SQ20).

The flow parameter analyzer 1122 in FIG. 2 obtains the time change in the flow parameter generated as described above for each of the OCTA image groups acquired at timings different from each other, for the designated pixel in the OCTA image or the VISTA image. Here, the OCTA image group is an example of a motion contrast data group. For example, the flow parameter analyzer 1122 can identify the time change in the flow parameters by sequentially identifying the flow parameters from the OCTA image groups acquired at each of times t = T1, T2, ....

According to the embodiments, the VISTA images of the object to be measured can be acquired merely with B-scans, such as line scans, without performing a three-dimensional OCT scan on the object to be measured. This can significantly reduce the amount of data required for VISTA while significantly shortening the measurement time. As a result, it is possible to acquire the measurement data required for VISTA in real time, to acquire the VISTA analysis results in real time, and/or to display the VISTA analysis results on the display apparatus 1300 in real time.

In addition, since VISTA processing can be performed using merely B-scans instead of three-dimensional scans, it is possible to repeatedly perform scans on the same location of the object to be measured. Thereby, it is possible to analyze the time change in the time variation.

Furthermore, since the VISTA analysis results can be acquired in real time, the time variation of the motion contrast intensity in the region of interest can be observed in detail while adjusting the interscan time, which is not taken into account in OCTA, in real time. For example, high and low blood flow velocity in the living eye can be depicted as the time variation of the motion contrast intensity. In this case, the vascular region where the blood flow velocity is fast can be observed in detail by shortening the interscan time, and the vascular region where the blood flow velocity is slow can be observed in detail by lengthening the interscan time.

It should be noted that the configuration of the analysis system 1000 according to the embodiments is not limited to the configuration shown in FIG. 1.

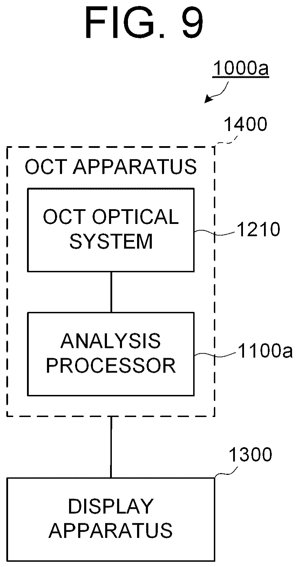

FIG. 9 shows a block diagram of another example of the configuration of the analysis system according to the embodiments. In FIG. 9, like reference numerals designate like parts as in FIG. 1. The same description may not be repeated.

An analysis system 1000a includes an OCT apparatus 1400 and the display apparatus 1300. The OCT apparatus 1400 has the functions of the analysis processing apparatus 1100 in the analysis system 1000. Specifically, the OCT apparatus 1400 includes an OCT optical system 1210 and an analysis processor 1100a. The OCT optical system 1210 is the OCT optical system included in the OCT apparatus 1200 in FIG. 1.

The analysis processor 1100a realizes the functions of the analysis processing apparatus 1100 in FIG. 1. The analysis processor 1100a has the same configuration as the analysis processing apparatus 1100 in FIG. 2. Here, the acquisition unit acquires the detection results of the interference light from the OCT optical system 1210 as measurement data, and forms OCT images based on the acquired measurement data. The acquisition unit sequentially forms three or more OCT images based on the measurement data. Here, the measurement data is acquired by repeatedly performing OCT B-scans three or more times on the same cross-section in the object to be measured using the OCT optical system 1210. Further, the acquisition unit forms the two or more pieces of motion contrast data (OCTA images) of the cross-section at interscan time intervals different from each other, from the formed three or more OCT images. The analyzer performs analysis processing for obtaining the time variation of the motion contrast intensity in the cross-section based on the two or more pieces of motion contrast data generated by the acquisition unit. The display controller causes the analysis results obtained by performing analysis processing executed by the analyzer to be displayed on the display apparatus 1300, in real time.

According to the analysis system 1000a shown in FIG. 9, the same effect as the analysis system 1000 shown in FIG. 1 can be obtained.

Next, an ophthalmic apparatus that realizes functions of an ophthalmic information processing apparatus, to which the analysis processing apparatus 1100 or the analysis processor 1100a according to the embodiments is applied, will be described. In other words, the ophthalmic information processing apparatus has the functions of the analysis processing apparatus 1100 or the analysis processor 1100a according to the embodiments, and the functions of the ophthalmic information processing apparatus are realized by an ophthalmic apparatus capable of performing OCT scan.

< Ophthalmic apparatus >

The ophthalmic apparatus according to the embodiments includes an ophthalmic imaging apparatus having at least the function of optical coherence tomography. The ophthalmic imaging apparatus included in the ophthalmic apparatus according to some embodiments further may also be configured to realize any one or more of functions, such as a fundus camera, a scanning laser ophthalmoscope, a slit lamp ophthalmoscope, or a surgical microscope, for example. The ophthalmic apparatus according to some embodiments includes any one or more of an ophthalmic measuring apparatus and an ophthalmic therapy apparatus, in addition to the ophthalmic imaging apparatus. The ophthalmic measuring apparatus included in the ophthalmic apparatus according to some embodiments is any one or more of an eye refractivity examination apparatus, a tonometer, a specular microscope, a wave-front analyzer, a perimeter, a microperimeter, and the like, for example. The ophthalmic therapy apparatus included in the ophthalmic apparatus according to some embodiments is any one or more of a laser therapy apparatus, a surgical apparatus, a surgical microscope, and the like, for example.

In the following embodiments, the ophthalmic apparatus includes the optical coherence tomography and the fundus camera. Although swept source OCT is employed as OCT, the type of OCT is not limited to the swept source OCT. It is also possible to employ other types of OCT (spectral domain OCT, time domain OCT, en-face OCT, or the like).

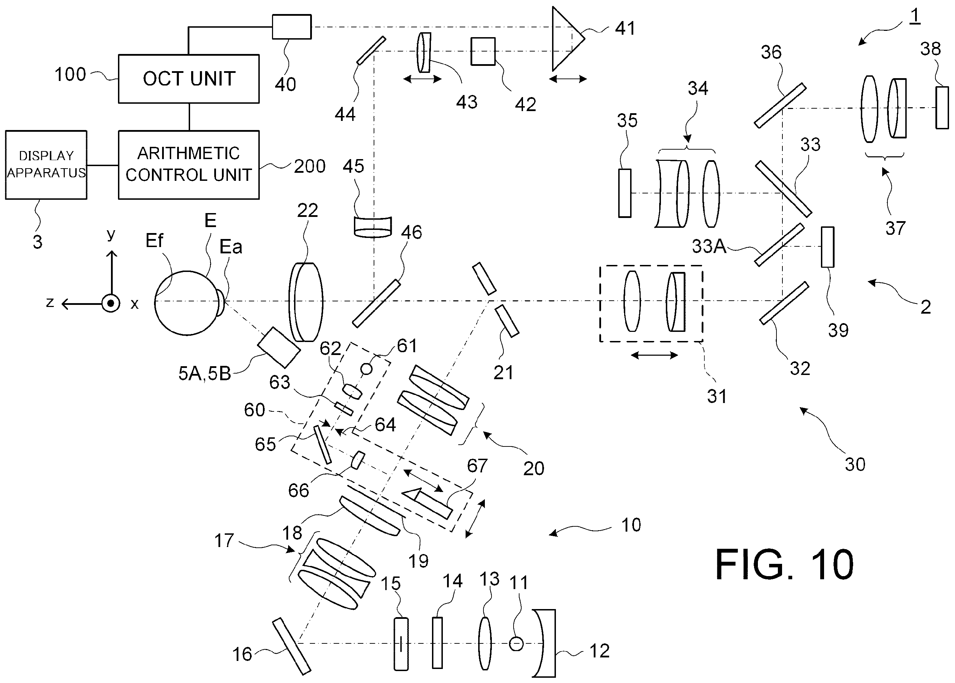

Hereinafter, it is assumed that the x-direction is a direction orthogonal to an optical axis direction of an objective lens (left-right direction), and the y-direction is a direction orthogonal to the optical axis direction of the objective lens (up-down direction). The z-direction is assumed to be the optical axis direction of the objective lens. It should be noted that, for convenience of explanation, the z-direction may represent the direction opposite to the z-direction in FIG. 10 in the following embodiments.

[Configuration]

[Optical system]

As shown in FIG. 10, the ophthalmic apparatus 1 according to the embodiments includes a fundus camera unit 2, an OCT unit 100, and an arithmetic control unit 200. The fundus camera unit 2 is provided with an optical system and a mechanism for acquiring front images of an eye E to be examined. The OCT unit 100 is provided with a part of an optical system and/or a mechanism for performing OCT. Other part of the optical system and the mechanism for performing OCT are provided in the fundus camera unit 2. The arithmetic control unit 200 includes one or more processors for performing various kinds of arithmetic processing and control processing. In addition to these elements, an arbitrary element or a unit, such as a member (chin rest, forehead pad, etc.) for supporting a face of the subject, a lens unit (for example, an attachment for an anterior segment OCT) for switching the target site of OCT, and the like, may be provided in the ophthalmic apparatus 1. Furthermore, the ophthalmic apparatus 1 includes a pair of anterior segment cameras 5A and 5B.

In some embodiments, the ophthalmic apparatus 1 includes a display apparatus 3. The display apparatus 3 displays a processing result (for example, an OCT image or the like) obtained by the arithmetic control unit 200, an image obtained by the fundus camera unit 2, operation guidance information for operating the ophthalmic apparatus 1, and the like.

[Fundus camera unit 2]

The fundus camera unit 2 is provided with an optical system for imaging (photographing) a fundus Ef of the eye E to be examined. An image (called fundus image, fundus photograph, etc.) of the fundus Ef to be acquired is a front image such as an observation image, a photographic image, or the like. The observation image is obtained by moving image shooting using near infrared light. The photographic image is a still image using or flash light. Furthermore, the fundus camera unit 2 can acquire the front image (anterior segment image) by photographing an anterior segment Ea of the eye E to be examined.

The fundus camera unit 2 includes an illumination optical system 10 and an imaging (photographing) optical system 30. The illumination optical system 10 irradiates illumination light onto the eye E to be examined. The imaging optical system 30 detects returning light of the illumination light from the eye E to be examined. Measurement light from the OCT unit 100 is guided to the eye E to be examined through an optical path in the fundus camera unit 2. Returning light of the measurement light is guided to the OCT unit 100 through the same optical path.

Light (observation illumination light) emitted from the observation light source 11 of the illumination optical system 10 is reflected by a reflective mirror 12 having a curved reflective surface, and becomes near-infrared light after being transmitted through a visible cut filter 14 via a condenser lens 13. Further, the observation illumination light is once converged near an imaging light source 15, is reflected by a mirror 16, and passes through relay lenses 17 and 18, a diaphragm 19, and a relay lens 20. Then, the observation illumination light is reflected on the peripheral part (surrounding area of a hole part) of a perforated mirror 21, is transmitted through a dichroic mirror 46, and is refracted by the objective lens 22, thereby illuminating the eye E to be examined (fundus Ef or anterior segment Ea). Returning light of the observation illumination light from the eye E to be examined is refracted by the objective lens 22, is transmitted through the dichroic mirror 46, passes through a hole part formed in the center area of the perforated mirror 21, travels through the photography focusing lens 31, and is reflected by the mirror 32. Further, this returning light is transmitted through a half mirror 33A, is reflected by a dichroic mirror 33, and forms an image on the light receiving surface of an image sensor 35 by a condenser lens 34. The image sensor 35 detects the returning light at a predetermined frame rate. It should be noted that the focus of the imaging optical system 30 is adjusted so as to coincide with the fundus Ef or the anterior segment Ea.

Light (imaging illumination light) emitted from the imaging light source 15 is irradiated onto the fundus Ef through the same route as that of the observation illumination light. Returning light of the imaging illumination light from the eye E to be examined is guided to the dichroic mirror 33 through the same route as that of the observation illumination light, is transmitted through the dichroic mirror 33, is reflected by a mirror 36, and forms an image on the light receiving surface of the image sensor 38 by a condenser lens 37.

An image (observation image) based on the fundus reflection light detected by the image sensor 35 is displayed on a display apparatus 3. It should be noted that when the imaging optical system 30 is focused on the anterior segment, an observation image of the anterior segment of the eye E to be examined is displayed. Furthermore, the display apparatus 3 displays an image (photographic image) based on the fundus reflection light detected by the image sensor 38. It should be noted that the display apparatus 3 for displaying the observation image and the display apparatus 3 for displaying the photographic image may be the same or different. When similar photographing is performed by illuminating the eye E to be examined with infrared light, an infrared photographic image is displayed.

A liquid crystal display (LCD) 39 displays a fixation target and a visual target used for visual acuity measurement. Part of light flux output from the LCD 39 is reflected by the half mirror 33A, is reflected by the mirror 32, travels through the photography focusing lens 31, and passes through the hole part of the perforated mirror 21. The light flux (beam) having passed through the hole part of the perforated mirror 21 is transmitted through the dichroic mirror 46, and is refracted by the objective lens 22, thereby being projected onto the fundus Ef.

By changing the display position of the fixation target on the screen of the LCD 39, the fixation position of the eye E to be examined can be changed. Examples of the fixation position include a fixation position for acquiring an image centered at a macula, a fixation position for acquiring an image centered at an optic disc, a fixation position for acquiring an image centered at a fundus center between the macula and the optic disc, a fixation position for acquiring an image of a site (fundus peripheral part) far away from the macula, and the like. The ophthalmic apparatus 1 according to some embodiments includes GUI (Graphical User Interface) and the like for designating at least one of such fixation positions. The ophthalmic apparatus 1 according to some embodiments includes GUI etc. for manually moving the fixation position (display position of the fixation target).

The configuration for presenting the movable fixation target to the eye E to be examined is not limited to the display device such LCD or the like. For example, the movable fixation target can be generated by selectively turning on a plurality of light sources of a light source array (light emitting diode (LED) array or the like). Alternatively, the movable fixation target can be generated using one or more movable light sources.

The focus optical system 60 generates a split indicator for adjusting the focus with respect to the eye E to be examined. The focus optical system 60 is movable along an optical path (illumination optical path) of the illumination optical system 10 in conjunction with the movement of the photography focusing lens 31 along an optical path (imaging optical path) of the imaging optical system 30. The reflection rod 67 can be inserted and removed into and from the illumination optical path. To perform focus adjustment, the reflective surface of the reflection rod 67 is arranged in a slanted position on the illumination optical path. Focus light emitted from an LED 61 passes through a relay lens 62, is split into two light beams by a split indicator plate 63, passes through a two-hole diaphragm 64, is reflected by a mirror 65, and is reflected after an image is once formed on the reflective surface of the reflection rod 67 by a condenser lens 66. Further, the focus light travels through the relay lens 20, is reflected by the perforated mirror 21, is transmitted through the dichroic mirror 46, and is refracted by the objective lens 22, thereby being projected onto the fundus Ef. Fundus reflection light of the focus light is guided to the image sensor 35 through the same route as the returning light of the observation illumination light. Manual focus or automatic focus can be performed based on the received light image (split indicator image) thereof.

The dichroic mirror 46 separates an optical path for OCT from an optical path for fundus photography. The dichroic mirror 46 reflects light of wavelength band used in OCT, and transmits light for fundus photography. The optical path for OCT (optical path of measurement light) is provided with, in order from the OCT unit 100 side to the dichroic mirror 46 side, a collimator lens unit 40, an optical path length changing unit 41, an optical scanner 42, an OCT focusing lens 43, a mirror 44, and a relay lens 45.

The optical path length changing unit 41 is movable in directions indicated by the arrow in FIG. 10, thereby changing the length of the optical path for OCT. This change in the optical path length is used for correcting the optical path length according to the axial length, adjusting the interference state, or the like. The optical path length changing unit 41 includes a corner cube and a mechanism for moving the corner cube.

The optical scanner 42 is disposed at a position optically conjugate to a pupil of the eye E to be examined. The optical scanner 42 deflects the measurement light LS traveling along the OCT optical path. The optical scanner 42 is a galvanometer scanner capable of scanning two-dimensionally, for example.

The OCT focusing lens 43 is moved along the optical path of the measurement light LS in order to perform focus adjustment of the optical system for OCT. The movement of the photography focusing lens 31, the movement of the focus optical system 60, and the movement of the OCT focusing lens 43 can be controlled in conjunction with each other.

[Anterior segment cameras 5A and 5B]

The anterior segment cameras 5A and 5B are used for obtaining relative position between the optical system of the ophthalmic apparatus 1 and the eye E to be examined in the same manner as the method disclosed in Japanese Unexamined Patent Application Publication No. 2013-248376, for example. The anterior segment cameras 5A and 5B are located on a surface of a housing (fundus camera unit 2 etc.) in which the optical system is stored to face the eye E to be examined. The ophthalmic apparatus 1 obtains the three-dimensional relative position between the optical system and the eye E to be examined, by analyzing two anterior segment images acquired substantially simultaneously from different directions by the anterior segment cameras 5A and 5B. The analysis of the two anterior segment images may be the same as the analysis disclosed in Japanese Unexamined Patent Application Publication No. 2013-248376. Furthermore, the number of anterior segment cameras may be any number equal to or more than two.

In the present examples, the position of the eye E to be examined (that is, the relative position between the eye E to be examined and the optical system) is obtained using two or more anterior segment cameras. However, a method of obtaining the position of the eye E to be examined is not limited to this. For example, the position of the eye E to be examined can be obtained by analyzing the front image (for example, the observation image of the anterior segment Ea) of the eye E to be examined. Alternatively, means for projecting an indicator onto a cornea of the eye E to be examined can be provided. Thereby, the position of the eye E to be examined can be obtained based on the projected position of this indicator (that is, the detection state of the corneal reflected light flux of this indicator).

[OCT unit 100]

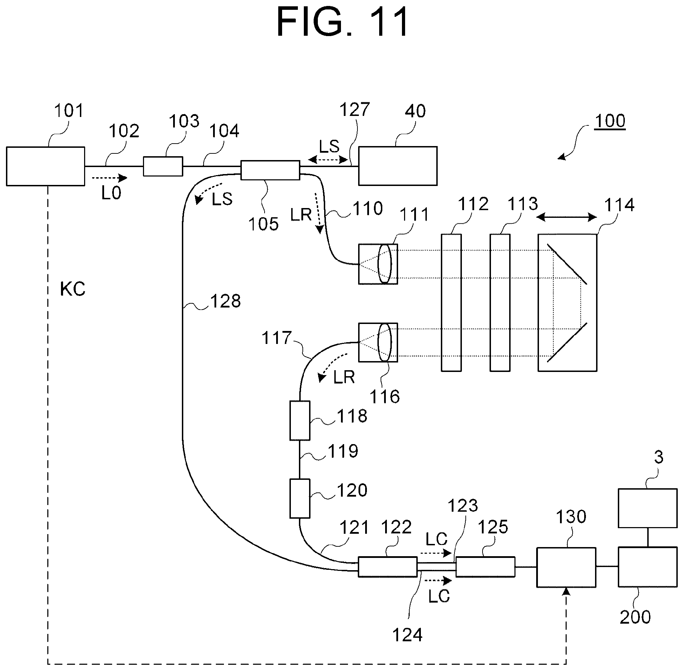

As illustrated by an example in FIG. 11, the OCT unit 100 is provided with an optical system for performing swept source OCT. This optical system includes an interference optical system. This interference optical system has a function that splits light from the wavelength tunable type (wavelength sweeping type) light source into measurement light and reference light, a function that makes the returning light of the measurement light from the eye E to be examined and the reference light having traveled through a reference optical path interfere with each other and generates interference light, and a function that detects the interference light. The detection result (detection signal) of the interference light obtained by the interference optical system is a signal indicating a spectrum of the interference light, and is sent to the arithmetic control unit 200.

The light source unit 101 includes a near-infrared tunable laser which changes the wavelength of the emitted light at high speed, for example. The light L0 emitted from the light source unit 101 is guided to the polarization controller 103 through the optical fiber 102, and the polarization state of the light L0 is adjusted. The light L0 whose polarization state has been adjusted is guided to the fiber coupler 105 through the optical fiber 104. The fiber coupler 105 splits the light L0 into the measurement light LS and the reference light LR.

The reference light LR is guided to the collimator 111 through the optical fiber 110. The reference light LR is converted into a parallel light flux by the collimator 111. Then, the reference light LR is guided to the corner cube 114 via an optical path length correction member 112 and a dispersion compensation member 113. The optical path length correction member 112 acts so as to match the optical path length of the reference light LR with the optical path length of the measurement light LS. The dispersion compensation member 113 acts so as to match the dispersion characteristics between the reference light LR and the measurement light LS. The corner cube 114 is movable in the incident direction of the reference light LR. With this, the optical path length of the reference light LR is changed.

The reference light LR that has traveled through the corner cube 114 passes through the dispersion compensation member 113 and the optical path length correction member 112, is converted from the parallel light flux to the convergent light flux by a collimator 116, and enters an optical fiber 117. The reference light LR that has entered the optical fiber 117 is guided to a polarization controller 118, and the polarization state of the reference light LR is adjusted. Then the reference light LR is guided to an attenuator 120 through an optical fiber 119, and the light amount of the reference light LR is adjusted. After that, the reference light LR is guided to a fiber coupler 122 through an optical fiber 121.

Meanwhile, the measurement light LS generated by the fiber coupler 105 is guided through an optical fiber 127, and is made into a parallel light beam by the collimator lens unit 40. The measurement light LS made into the parallel light beam travels through the optical path length changing unit 41, the optical scanner 42, the OCT focusing lens 43, the mirror 44, and the relay lens 45. The measurement light LS having traveled through the relay lens 45 is reflected by the dichroic mirror 46, is refracted by the objective lens 22, and is irradiated onto the eye E to be examined. The measurement light LS is scattered and reflected at various depth positions of the eye E to be examined. Returning light of the measurement light LS from the eye E to be examined advances in the same path as the forward path in the opposite direction, is guided to the fiber coupler 105, and then reaches the fiber coupler 122 via the optical fiber 128. It should be noted that the incident end of the optical fiber 127, into which the measurement light LS enters, is arranged at a position substantially optically conjugate to the fundus Ef of the eye E to be examined.

The fiber coupler 122 combines (interferes) the measurement light LS incident through the optical fiber 128 and the reference light LR incident through the optical fiber 121 to generate interference light. The fiber coupler 122 splits the interference light at a predetermined splitting ratio (e.g., 1: 1) to generate a pair of interference light LC. The pair of interference light LC is guided to a detector 125 through optical fibers 123 and 124, respectively.

The detector 125 is a balanced photodiode, for example. The balanced photodiode includes a pair of photodetectors in which each photodiode detects each of the pair of interference light LC. The balanced photodiode outputs the difference between a pair of detection results acquired by the pair of photodetectors. The detector 125 sends the output (detection signal) to a DAQ (Data Acquisition System) 130.

A clock KC is supplied from the light source unit 101 to the DAQ 130. The clock KC is generated in the light source unit 101 in synchronization with the output timing of each wavelength within a predetermined wavelength range performed by the wavelength tunable type light source. For example, the light source unit 101 optically delays one of the two pieces of branched light obtained by branching the light L0 of each output wavelength, and then generates the clock KC based on the result of the detection of the combined light of the two pieces of branched light. The DAQ 130 performs sampling the detection signal input from the detector 125 based on the clock KC. The DAQ 130 sends the sampling result of the detection signal from the detector 125 to the arithmetic control unit 200.

In the present examples, both the optical path length changing unit 41 that changes the length of the optical path of the measurement light LS (i.e., measurement optical path or measurement arm) and the corner cube 114 that changes the length of the optical path of the reference light LR (i.e., reference optical path or reference arm) are provided. However, any one of the optical path length changing unit 41 and the corner cube 114 may be provided. Alternatively, the difference between the measurement optical path length and the reference optical path length can be changed using other optical members.

[Processing system]

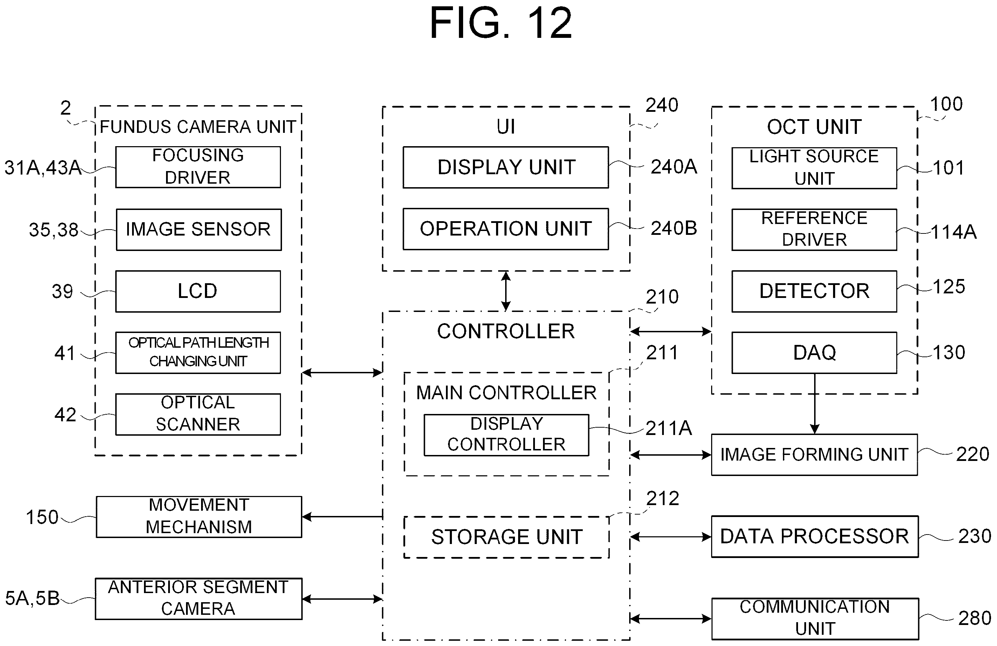

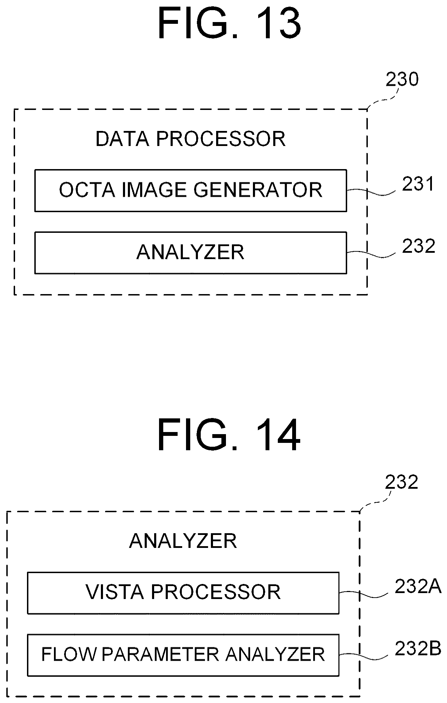

FIG. 12, FIG. 13, and FIG. 14 show examples of a configuration of a processing system (control system) of the ophthalmic apparatus 1. In FIG. 12, FIG. 13, and FIG. 14, some of components included in the ophthalmic apparatus 1 are omitted. FIG. 12 represents an example of a functional block diagram illustrating the processing system of the ophthalmic apparatus 1. In FIG. 12, like reference numerals designate like parts as in FIG. 10 and FIG. 11. The same description may not be repeated. FIG. 13 represents an example of a functional block diagram of a data processor 230 in FIG. 12. FIG. 14 represents an example of a functional block diagram of an analyzer 232 in FIG. 13.

A controller 210, an image forming unit 220, and a data processor 230, which are shown in FIG. 12, are provided, for example, in the arithmetic control unit 200.

(Controller 210)

The controller 210 executes various controls. The controller 210 includes a main controller 211 and a storage unit 212.

(Main controller 211)

The main controller 211 includes a processor (for example, control processor), and controls each part (including each element show in FIGs. 10 to 14) of the ophthalmic apparatus 1. For example, the main controller 211 controls each part of the optical system of the fundus camera unit 2 shown in FIG. 10 and FIG. 11, each part of the optical system of the OCT unit 100, the anterior segment cameras 5A and 5B, a movement mechanism 150 that moves the optical system described above, the image forming unit 220, the data processor 230, and a user interface (UI) 240.

Examples of control for the fundus camera unit 2 include control for the focusing drivers 31A and 43A, control for the image sensors 35 and 38, control for the LCD 39, control for the optical path length changing unit 41, and control for the optical scanner 42.

Examples of control for the focusing driver 31A include control of moving the photography focusing lens 31 in the optical axis direction. Examples of control for the focusing driver 43A include control of moving the OCT focusing lens 43 in the optical axis direction.

Examples of control for the image sensors 35 and 38 include control of the light receiving sensitivity to the imaging elements, control of the frame rate (light receiving timing, exposure time), and readout control of the light receiving result for the imaging elements.

Examples of control for the LCD 39 include control of a fixation position. For example, the main controller 211 controls the LCD 39 to display the fixation target at a position on the screen of the LCD 39 corresponding to the fixation position set manually or automatically. Moreover, the main controller 211 can change the display position of the fixation target displayed on the LCD 39 (in a continuous manner or in a phased manner). Thereby, the fixation target can be moved (that is, the fixation position can be changed). The display position of the fixation target and movement mode of the fixation target are set manually or automatically. Manual setting is performed using GUI, for example. Automatic setting is performed by the data processor 230, for example.

Examples of control for the optical path length changing unit 41 include control of changing an optical path length of the measurement light LS. The main controller 211 moves the optical path length changing unit 41 along an optical path of the measurement light LS to change the optical path length of the measurement light LS, by controlling a driver that drives the corner cube of the optical path length changing unit 41.

Examples of the control for the optical scanner 42 include control of a scan mode, a scan range (scan start position, scan end position) including scan position, and a scan speed. The main controller 211 can perform OCT scan with the measurement light LS on a desired region in a measurement site (imaging site) by performing control for the optical scanner 42.

Further, the main controller 211 can control the observation light source 11, the imaging light source 15, the focus optical system 60, and the like.

Examples of the control for the OCT unit 100 include control for the light source unit 101, control for the reference driver 114A, control for the detector 125, control for the DAQ 130, and the like.

Examples of the control for the light source unit 101 include controlling of turning the light source on and off, control of the light quantity of the light emitted from the light source, control of the wavelength sweep range, wavelength sweep speed, and control of the timing of emission of light with each wavelength component.

Examples of control for the reference driver 114A include control of changing the optical path length of the reference light LR. The main controller 211 moves the corner cube 114 along an optical path of the reference light LR to change the optical path length of the reference light LR, by controlling the reference driver 114A.

Examples of control for the detector 125 include control of light receiving sensitivity to the detecting elements, control of frame rate (light receiving timing), and readout control of the light receiving result for the detecting elements.

Examples of control for the DAQ 130 include control of capturing (capturing timing, sampling timing) of the detection result of the interference light obtained by the detector 125, and readout control of the interference signal corresponding to the detection result of the captured interference light.

Examples of the control for the anterior segment cameras 5A and 5B include control of the light receiving sensitivity of each camera, control of the frame rate (light receiving timing), and synchronization control of the anterior segment cameras 5A and 5B.

The movement mechanism 150 three-dimensionally moves at least the fundus camera unit 2 (optical system), for example. In a typical example, the movement mechanism 150 includes a mechanism for moving at least the fundus camera unit 2 in the x-direction (left-right direction), a mechanism for moving at least the fundus camera unit 2 in the y-direction (up-down direction), and a mechanism for moving at least the fundus camera unit 2 in the z-direction (depth direction, front-back direction). The mechanism for moving in the x-direction includes an x-stage movable in the x-direction and an x-movement mechanism for moving the x-stage, for example. The mechanism for moving in the y-direction includes a y-stage movable in the y-direction and a y-movement mechanism for moving the y-stage, for example. The mechanism for moving in the z-direction includes a z-stage movable in the z-direction and a z-movement mechanism for moving the z-stage, for example. Each movement mechanism includes a pulse motor as an actuator and operates under the control from the main controller 211.

The control for the movement mechanism 150 is used for alignment and/or tracking. Here, tracking is to move the optical system of the apparatus according to the eye movement of the eye E to be examined. To perform tracking, alignment and focus adjustment are performed in advance. The tracking is a function of maintaining a suitable positional relationship in which alignment and focusing are matched by causing the position of the optical system of the apparatus and the like to follow the eye movement. In some embodiments, the movement mechanism 150 is configured to be controlled to change the optical path length of the reference light (that is, the difference of the optical path length between the optical path of the measurement light and the optical path of the reference light).

In the case of manual alignment, a user operates the UI 240 to relatively move the optical system and the eye E to be examined so as to cancel the displacement of the eye E to be examined relative to the optical system. For example, the main controller 211 controls the movement mechanism 150 to relatively move the optical system relative to the eye E to be examined, by outputting a control signal corresponding to the operation content for the UI 240 to the movement mechanism 150.

In the case of automatic alignment, the main controller 211 controls the movement mechanism 150 to relatively move the optical system relative to the eye E to be examined so as to cancel the displacement of the eye E to be examined relative to the optical system, by outputting a control signal to the movement mechanism 150. Specifically, the main controller 211 performs arithmetic processing using a trigonometry based on the positional relationship between the anterior segment cameras 5A and 5B and the eye E to be examined, and controls the movement mechanism 150 so that the positional relationship of the eye E to be examined relative to the optical system becomes a predetermined relationship. Such processing is disclosed in, for example, Japanese Unexamined Patent Application Publication No. 2013-248376. In some embodiments, the main controller 211 controls the movement mechanism 150 to relatively move the optical system relative to the eye E to be examined so that the optical axis of the optical system approximately coincides with an axis (visual axis) of the eye E to be examined and the distance of the optical system with respect to the eye E to be examined is a predetermined working distance. Here, the working distance is a preset value which is called a working distance of the objective lens 22, and it means the distance between the eye E to be examined and the optical system when measuring (imaging) using the optical system.

Further, the main controller 211 includes a display controller 211A. The display controller 211A can display various kinds of information on a display unit 240A. For example, the display controller 211A causes the fundus image or the anterior segment image acquired using the fundus camera unit 2, the OCT image or the OCTA image acquired using the OCT unit 100, and/or the data processing result(s) obtained by the data processor 230 described below to be displayed on the display unit 240A. Examples of the OCT image acquired using the OCT unit 100 include a B-scan image, a projection image, and an en-face an image. Examples of the data processing result(s) obtained by the data processor 230 include VISTA analysis result(s), the VISTA image, and the image representing the time change in the flow parameter(s). In some embodiments, the display controller 211A displays the data processing result(s) in association with at least one of the fundus image (or anterior segment image) or the OCT image on the display unit 240A.

(Storage unit 212)

The storage unit 212 stores various types of data. The function of the storage unit 212 is realized by a memory device such as a memory or storage device. Examples of the data stored in the storage unit 212 include image data of the fundus image, image data of the anterior segment image, OCT data (including the OCT image), OCTA data (motion contrast data), VISTA analysis result, and information on eye to be examined. The information on the eye to be examined includes information on the examinee such as patient ID and name, and information on the eye to be examined such as identification information of the left/right eye or an electronic health record. The storage unit 212 further may store various types of programs for executing various processors (control processor, image forming processor, data processing processor).

(Image forming unit 220)

The image forming unit 220 includes a processor (image forming processor, for example) and forms the OCT image of the eye E to be examined based on the output (sampling result(s) of the detection signals) from the DAQ 130. For example, the image forming unit 220 forms a reflection intensity profile for each A-line by performing signal processing on the spectral distribution on the basis of the sampling detection for each A-line in the same manner as in the conventional swept source OCT, images these A-line profiles, and arranges them along the scan line. The above signal processing includes noise removal (noise reduction), filtering, fast Fourier transform (FFT), and the like. In the case of performing OCT of another type, the image forming unit 220 performs known processing according to the performed type.

(Data processor 230)

The data processor 230 includes a processor (data processing processor, for example) and performs various kinds of image processing and various kinds of analysis processing on the image formed by the image forming unit 220. At least two of the processor included in the main controller 211, the processor included in the data processor 230, or the processor included in the image forming unit 220 may be configured by a single processor.

The data processor 230 performs known image processing such as interpolation for interpolating pixels between tomographic images to form image data of the three-dimensional image of the fundus Ef or the anterior segment Ea. It should be noted that the image data of the three-dimensional image means image data in which the positions of pixels are defined in a three-dimensional coordinate system. Examples of the image data of the three-dimensional image include image data defined by voxels three-dimensionally arranged. Such image data is referred to as volume data or voxel data. When displaying an image based on volume data, the data processor 230 performs rendering processing (volume rendering, maximum intensity projection (MIP), etc.) on the volume data, thereby forming image data of a pseudo three-dimensional image viewed from a particular visual line. The pseudo three-dimensional image is displayed on the display device such as the display unit 240A.

Further, stack data of a plurality of tomographic images may be formed as the image data of the three-dimensional image. The stack data is image data formed by three-dimensionally arranging tomographic images along a plurality of scan lines based on positional relationship of the scan lines. That is, the stack data is image data obtained by representing tomographic images, which are originally defined in their respective two-dimensional coordinate systems, by a single three-dimensional coordinate system. That is, the stack data is image data formed by embedding tomographic images into a single three-dimensional space.

In some embodiments, the data processor 230 generates a B-scan image by arranging the A-scan images in a B-scan direction. In some embodiments, the data processor 230 can form a B-mode image (B-scan image) (longitudinal cross-sectional image, axial cross-sectional image) in an arbitrary cross section, a C-mode image (C-scan image) (transverse section image, horizontal cross-sectional image) in an arbitrary cross section, a projection image, a shadowgram, etc., by performing various renderings on the acquired three-dimensional data set (volume data, stack data, etc.). An image in an arbitrary cross section such as the B-scan image or the C-scan image is formed by selecting pixels (voxels) on a designated cross section from the three-dimensional OCT data. The projection image is formed by projecting the three-dimensional data set in a predetermined direction (z-direction, depth direction, axial direction). The shadowgram is formed by projecting a part of the three-dimensional data set in a predetermined direction. Examples of the part of the three-dimensional data set include partial data corresponding to a specific layer. By changing the depth range in a layer direction to be integrated, the two or more shadowgrams differ from each other can be formed. An image having a viewpoint on the front side of the eye to be examined, such as the C-scan image, the projection image, and the shadowgram, is called a front image (en-face image).

Further, the data processor 230 can obtain a three-dimensional position of the eye E to be examined based on the positions of the anterior segment cameras 5A and 5B and a position of a characteristic site.

In this case, the data processor 230, first, analyzes, as a characteristic site identifying unit, each photographic images captured by the anterior segment cameras 5A and 5B to identify a position (called characteristic position) in the photographic image corresponding to the characteristic site of the anterior segment Ea. Examples of the characteristic site includes a pupil region of the eye E to be examined, a center position of the pupil of the eye E to be examined, a position of the center of gravity of the pupil, a center position of the cornea, a position of the corneal apex, a center position of the eye to be examined, and an iris. For example, the data processor 230 identifies an image region (pupil region) corresponding to the pupil of the eye E to be examined, based on the distribution of pixel values (luminance values etc.) of the photographic image.

Next, the data processor 230 identifies the center position of the identified pupil region. As described above, the pupil is substantially circular; therefore, it is possible to identify the contour of the pupil region, to identify the center position of this contour (an approximate circle or an approximate ellipse thereof), and to treat this as the pupil center position. Instead, by obtaining the center of gravity of the pupil region, this position may be used as the position of the center of gravity of the pupil. The data processor 230 can sequentially identify the characteristic positions corresponding to the characteristic site for the photographic images sequentially obtained by the anterior segment cameras 5A and 5B. It should be noted that the data processor 230 may identify the characteristic positions for the photographic images sequentially obtained by the anterior segment cameras 5A and 5B every any number (one or more) of frames.

Then, the data processor 230 identifies, as a three-dimensional position calculator, the three-dimensional position of the characteristic site as a three-dimensional position of the eye E to be examined, based on the positions of the anterior segment cameras 5A and 5B and the characteristic positions corresponding to the identified characteristic site. The data processor 230 calculates the three-dimensional position of the eye E to be examined by applying known trigonometry to the positions of the two anterior segment cameras 5A and 5B (these are known) and the positions corresponding to the characteristic site in two photographic images. The obtained three-dimensional position is sent to the main controller 211. The main controller 211 relatively moves the optical system relative to the eye E to be examined to perform position matching of the optical system with respect to the eye E to be examined, by controlling the movement mechanism 150 based on the obtained three-dimensional position. In other words, the main controller 211 controls the movement mechanism 150 based on the three-dimensional position so that the position of the optical axis of the optical system in the x-direction and the y-direction respectively coincide with the position of the three-dimensional position in the x-direction and the y-direction and the distance in the z-direction becomes a predetermined working distance.

As shown in FIG. 13, the data processor 230 further includes an OCTA image generator 231, and an analyzer 232.

The OCTA image generator 231 can build (form) the OCTA image (blood vessel emphasized image, angiogram) in which retinal blood vessels and choroidal blood vessels are emphasized (highlighted), based on data (for example, B-scan image data) acquired in time series by performing OCT scan. The OCTA image may be a tomographic image or a front image.

In some embodiments, the OCTA image generator 231 compares the two B-scan images acquired by repeatedly performing B-scans on the approximately same site of the eye E to be examined, and converts the pixel value of a change portion of the signal intensity into a pixel value corresponding to a change amount in the change portion to build the emphasized image in which the change portion is emphasized. Further, the data processor 230 can form the OCTA (angiography) image by extracting information of a predetermined thickness amount at a desired site from a plurality of built emphasized images and building as an en-face image.

In the present embodiment, the OCTA image generator 231 generates the two or more OCTA images of the cross-section at interscan time intervals different from each other. Here, the two or more OCTA images are acquired by repeatedly performing B-scans three or more times on the same cross-section in the eye E to be examined (for example, fundus Ef).

The analyzer 232 has the functions of the analyzer 1120 in FIG. 2. In other words, the analyzer 232 performs VISTA processing using two or more OCTA images, which are generated by the OCTA image generator 231, at interscan time intervals different from each other, identifies the flow parameters, and obtains the time change in the flow parameters.

The analyzer 232 includes, as shown in FIG. 14, a VISTA processor 232A and a flow parameter analyzer 232B.

The VISTA processor 232A has the functions of the VISTA processor 1121 in FIG. 2. In other words, the VISTA processor 232A has the same configuration as the VISTA processor 1121 shown in FIG. 5. The VISTA processor 232A executes the VISTA processing using the two or more OCTA images generated by the OCTA image generator 231, and identifies the time variation of the motion contrast intensity in the cross-section. The VISTA processor 232A can generate the VISTA image representing the distribution of the identified time variation of the motion contrast intensity as an analysis result. Specifically, the VISTA processor 232A generates the image representing the time variation of the motion contrast intensity, based on a saturation time of the motion contrast intensity at each of a plurality of positions in the cross-section of a measured site (for example, fundus Ef) of the eye E to be examined.

The flow parameter analyzer 232B has the functions of the flow parameter analyzer 1122 in FIG. 2. In other words, the flow parameter analyzer 232B generates the information representing the time change in the time variation of the motion contrast intensity at a designated position in the cross-section on which the OCT scans are repeatedly performed. Specifically, the flow parameter analyzer 1122 identifies the time variation of the motion contrast intensity at the same designated position in respective images of the two or more VISTA images acquired at timings different from each other, as the flow parameter. Further, the flow parameter analyzer 1122 generates the information representing the time change in the identified flow parameters.

In addition, the analyzer 232 performs predetermined processing using the detection result(s) of the interference light LC, the OCT image, or the OCTA image.

For example, the analyzer 232 analyzes the detection result(s) of the interference light LC, the OCT image, or the OCTA image to output evaluation value(s) (including statistical value(s) of the evaluation values) corresponding to the image quality (signal-to-noise ratio) of the OCT image or the OCTA image as an analysis result. The main controller 211 can control at least one of the focusing driver 43A, the optical path length changing unit 41, the polarization controller 103, or the polarization controller 118 based on the analysis result(s) obtained by the analyzer 232.

For example, the analyzer 232 analyzes the detection result(s) of the interference light LC obtained by performing OCT measurement to determine a focus state of the measurement light LS in the fine focus adjustment control. For example, the main controller 211 repeatedly performs OCT measurements while controlling the focusing driver 43A according to a predetermined algorithm. The analyzer 232 analyzes the detection results of the interference light LC repeatedly acquired by performing OCT measurement to calculate a predetermined evaluation value relating to the image quality of the OCT image (or the OCTA image). The analyzer 232 determines whether or not the calculated evaluation value is equal to or less than a threshold value. In some embodiments, the fine focus adjustment is continued until the calculated evaluation value becomes equal to or less than the threshold value. That is, when the evaluation value is equal to or less than the threshold value, it is determined that the focus state of the measurement light LS is appropriate. And the fine focus adjustment is continued until it is determined that the focus state of the measurement light LS is appropriate.

In some embodiments, the main controller 211 monitors the intensity of the interference signal (interference intensity, interference sensitivity) acquired sequentially while acquiring the interference signal by performing the repetitive OCT measurements as described above. In addition, while performing this monitoring process, the OCT focusing lens 43 is moved to find the position of the OCT focusing lens 43 in which the interference intensity is maximized. With the fine focus adjustment thus performed, the OCT focusing lens 43 can be guided to the position where the interference intensity is optimized.

Further, the analyzer 232 analyzes the detection result of the interference light LC obtained by performing OCT measurement to determine a polarization state of at least one of the measurement light LS or the reference light LR. For example, the main controller 211 repeatedly performs the repetitive OCT measurements while controlling at least one of the polarization controllers 103 or 118 according to a predetermined algorithm. In some embodiments, the main controller 211 controls the attenuator 120 to change an attenuation of the reference light LR. The analyzer 232 analyzes the detection results of the interference light LC repeatedly acquired by performing OCT measurement to calculate a predetermined evaluation value relating to the image quality of the OCT image (or the OCTA image). The analyzer 232 determines whether or not the calculated evaluation value is equal to or less than a threshold value. The threshold value is set in advance. Polarization adjustment is continued until the calculated evaluation value becomes equal to or less than the threshold value. That is, when the evaluation value is equal to or less than the threshold value, it is determined that the polarization state of the measurement light LS is appropriate. And the polarization adjustment is continued until it is determined that the polarization state of the measurement light LS is appropriate.

In some embodiments, the main controller 211 can monitor the interference intensity also in the polarization adjustment.

Furthermore, the analyzer 232 can perform predetermined analysis processing on the OCT image or the OCTA image. Examples of the analysis processing for the OCT image include identification of a predetermined site (tissue, lesion) of the eye E to be examined: calculation of a distance, area, angle, ratio, or density between designated sites (distance between layers, interlayer distance); calculation by a designated formula; identification of the shape of the predetermined site; calculation of these statistics; calculation of distribution of the measured value or the statistical values; image processing based on these analysis processing results, and the like. Examples of the predetermined tissue include a blood vessel, an optic disc, a fovea, a macula, and the like. Examples of the predetermined lesion include a leukoma, a hemorrhage, and the like. Examples of the analysis processing for the OCTA image include identification blood vessel wall, identification of the vascular region, identification of a connection relationship between two or more vascular region, identification of a distribution of vascular region, identification of blood flow, calculation of blood flow velocity, and determination of artery/vein.

(User interface 240)

As shown in FIG. 12, the user interface 240 includes the display unit 240A and an operation unit 240B. The display unit 240A includes the display apparatus 3. The operation unit 240B includes various operation devices and input devices.

The user interface 240 may include a device having the output function and the input function integrated together, such as a touch panel display, for example. In another embodiment, at least a part of the user interface 240 may not be included in the ophthalmic apparatus. For example, the display device may be an external device connected to the ophthalmic apparatus.

(Communication unit 280)

The communication unit 280 shown in FIG. 12 has the functions of communicating with an external device (not shown). The communication unit 280 includes a communication interface according to the mode of communication with the external device. Examples of the external device include a server apparatus, an OCT apparatus, a scanning optical ophthalmoscope, a slit lamp ophthalmoscope, an ophthalmic measuring apparatus, and an ophthalmic treatment apparatus. Examples of the ophthalmic measuring apparatus include an eye refractivity examination apparatus, a tonometer, a specular microscope, a wave-front analyzer, a perimeter, and a micro perimeter. Examples of the ophthalmic treatment apparatus include a laser treatment apparatus, a surgical apparatus, and a surgical microscope. Alternatively, the external device may also be a device (reader) having the function of reading information from a recording medium or a device (writer) having the function of writing information to a recording medium. Further, the external device may be a hospital information system (HIS) server, a Digital Imaging and Communications in Medicine (DICOM) server, a doctor terminal, a mobile terminal, a personal terminal, a cloud server, or the like.

The arithmetic control unit 200 (controller 210, image forming unit 220, and data processor 230) is an example of the "analysis processing apparatus" according to the embodiments. The optical system from the objective lens 22 to the OCT unit 100 is an example of the "OCT optical system" according to the embodiments. The OCTA image is an example of the "motion contrast data" according to the embodiments. The VISTA image is an example of the "image representing a time variation of motion contrast intensity" according to the embodiments. The display unit 240A (display apparatus 3) is an example of the "display means" according to the embodiments.

(Operation)

An example of an operation of the ophthalmic apparatus 1 according to the embodiments will be described.

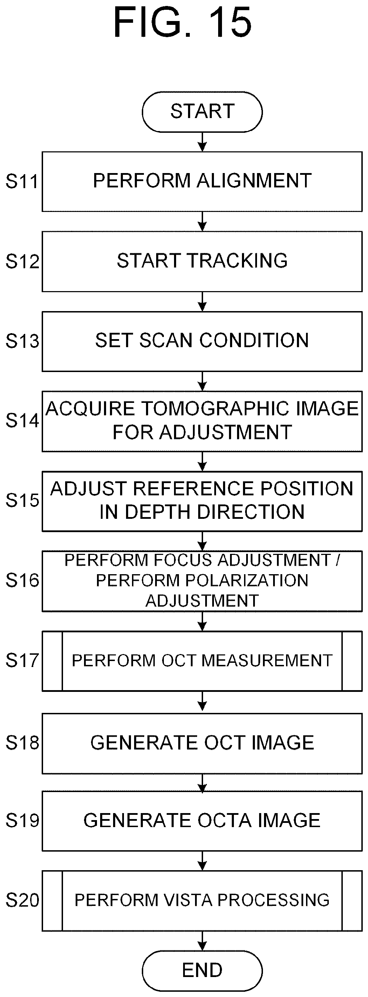

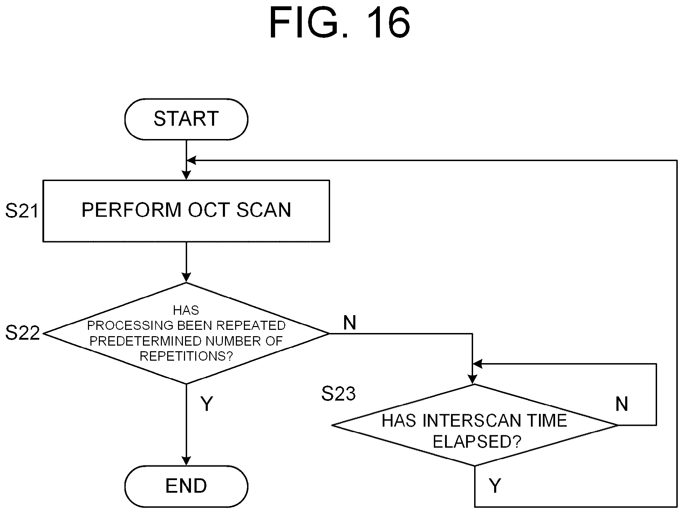

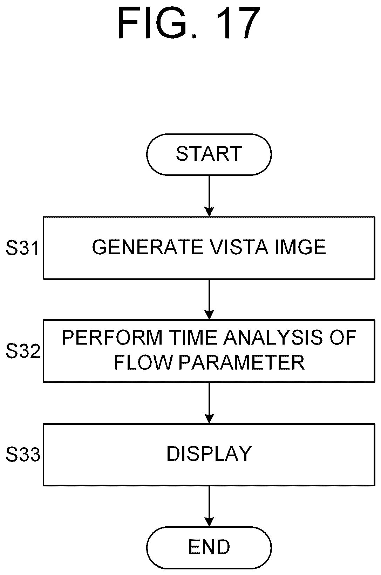

FIG. 15, FIG. 16, and FIG. 17 show examples of the operation of the ophthalmic apparatus 1 according to the embodiments. FIG. 15 represents a flowchart of an example of the operation of the ophthalmic apparatus 1. FIG. 16 represents a flowchart of an example of the operation of the ophthalmic apparatus 1, which performs processing of step S17 in FIG. 15. FIG. 17 represents a flowchart of an example of the operation of the ophthalmic apparatus 1, which performs processing of step S20 in FIG. 15. The storage unit 212 stores computer programs for realizing the processing shown in FIGs. 15 to 17. The main controller 211 operates according to the computer programs, and thereby the main controller 211 performs the processing shown in FIGs. 15 to 17.

(S11: Perform alignment)

First, the main controller 211 performs alignment.

For example, the main controller 211 controls the LCD 39 to present the fixation target for alignment to the eye E to be examined. The main controller 211 controls the anterior segment cameras 5A and 5B to substantially simultaneously photograph the anterior segment Ea of the eye E to be examined. The data processor 230 receives control from the main controller 211, analyzes a pair of the anterior segment images acquired substantially simultaneously by the anterior segment cameras 5A and 5B to identify a pupil center position of the eye E to be examined as the characteristic site. The data processor 230 obtains the three-dimensional position of the eye E to be examined by performing arithmetic processing using a trigonometric method based on the positional relationship between the pair of the anterior segment cameras 5A and 5B and the eye E to be examined.

The main controller 211 controls the movement mechanism 150 based on the three-dimensional position of the eye E to be examined, which is obtained by the data processor 230, so that the optical system (for example, the fundus camera unit 2) and the eye E to be examined have a predetermined positional relationship. Here, the predetermined positional relationship is a positional relationship in which photographing and inspection of the eye E to be examined can be performed using the optical system.

(S12: Start tracking)

Subsequently, the main controller 211 starts tracking.

For example, the main controller 211 controls the movement mechanism 150 so that the predetermined positional relationship between the eye E to be examined and the optical system is maintained by the alignment performed in step S11. In some embodiments, the main controller 211 controls the movement mechanism 150 based on the anterior segment images acquired by the anterior segment cameras 5A or 5B to start tracking control. This tracking control is continued until the OCTA measurement is completed.

(S13: Set scan condition)

Next, the main controller 211 sets the scan condition(s) of OCT scan to the storage unit 212. The main controller 211 controls the optical scanner 42 according to the scan condition(s) set to the storage unit 212 to perform OCT scan.

Examples of the scan condition include the scan mode, the scan range (scan start position, scan end position) including scan position, the scan speed, the interscan time, and the number of repetitions of scan for performing the VISTA processing. In the present embodiment, the line scan mode is set as the scan mode.

(S14: Acquire tomographic image for adjustment)

The main controller 211 controls the LCD 39 to display the fixation target for OCT measurement at a predetermined position on the LCD 39. The main controller 211 can display the fixation target at a display position on the LCD 39 corresponding to a position of an optical axis of the optical axis on the fundus Ef.

Subsequently, the main controller 211 controls the OCT unit 100 to perform OCT provisional measurement, and to acquire a tomographic image for adjusting a reference position of the measurement range in the depth direction. Specifically, the main controller 211 controls the optical scanner 42 to deflect the measurement light LS generated based on the light L0 emitted from the light source unit 101 and to scan fundus Ef of the eye E to be examined with the deflected measurement light LS. The detection result of the interference light obtained by scanning with the measurement light LS is sent to the image forming unit 220 after being sampled in synchronization with the clock KC. The image forming unit 220 forms the tomographic image (OCT image) of the eye E to be examined from the obtained interference signals.

(S15: Adjust reference position in depth direction)

Subsequently, the main controller 211 adjusts the reference position of the measurement range in the depth direction (z-direction).

For example, the main controller 211 controls the data processor 230 to identify a predetermined site (for example, sclera) in the tomographic image acquired in step S14, and sets a position separated by a predetermined distance in the depth direction from the identified position of the predetermined site as the reference position of the measurement range. The main controller 211 controls at least one of the optical path length changing units 41 or 114 according to the reference position. Alternatively, a predetermined position determined in advance so that the optical path lengths of the measurement light LS and the reference light LR substantially coincide may be set as the reference position of the measurement range.

(S16: Adjust focusing, adjust polarization)

Next, the main controller 211 performs control of adjusting focusing and of adjusting polarization.

For example, the main controller 211 controls the OCT unit 100 to perform OCT measurement, after controlling the focusing driver 43A to move the OCT focusing lens 43 by a predetermined distance. The main controller 211 controls the data processor 230 to determine the focus state of the measurement light LS based on the detection result of the interference light acquired by the OCT measurement, as described above. When it is determined that the focus state is not appropriate based on the determination result of the data processor 230, the main controller 211 controls the focusing driver 43A again and repeats this until it is determined that the focus state of the measurement light LS is appropriate.

Further, for example, the main controller 211 controls the OCT unit 100 to perform OCT measurement after controlling at least one of the polarization controllers 103 or 118 to change the polarization state of at least one of the light L0 or the measurement light LS by a predetermined amount. The main controller 211 controls the image forming unit 220 to form the OCT image on the basis of the acquired detection results of the interference light. The main controller 211 controls the data processor 230 to determine the image quality of the OCT image acquired by the OCT measurement, as described above. When it is determined that the polarization state is not appropriate based on the determination result of the data processor 230, the main controller 211 controls the polarization controllers 103 and 118 again and repeats this until it is determined that the polarization state of the measurement light LS is appropriate.

In some embodiments, step S13 is performed after step S14 and step S15.

(S17: Perform OCT measurement)

Subsequently, the main controller 211 controls the OCT unit 100, etc. according to the scan condition(s) set in step S13 in a state where the optical system is set in step S14 and step S15. Thereby, the line scans are repeatedly performed on the fundus Ef at predetermined interscan time intervals. In other words, a plurality of pieces of OCT data acquired at predetermined interscan time intervals for the same cross-section of the fundus Ef is obtained. The details of step S17 will be described below.

(S18: Form OCT image)

Next, the main controller 211 controls the image forming unit 220 to sequentially form the B-scan images (OCT images) based on the respective plurality of pieces of OCT data acquire in step S17. Thereby, the B-scan images IMG1 to IMG7 shown in FIG. 3 or FIG. 4 are sequentially formed, for example.

In some embodiments, step S17 and step 18 are performed in parallel.

(S19: Generate OCTA image)

Subsequently, the main controller 211 controls the OCTA image generator 231 to generate a plurality of OCTA images of the cross-section at interscan time intervals different from each other, from the plurality of OCT images formed in step S18. With this, the plurality of OCTA image groups can be acquired. A first OCTA image group among the OCTA image groups includes, for example, the OCTA images IMA11 to IMA 14 shown in FIG. 3, and a second OCTA image group includes, for example, OCTA images IMA21 to IMA24 shown in FIG. 4.

(S20: Perform VISTA processing)

Subsequently, the main controller 211 controls the analyzer 232 to execute the VISTA processing using the plurality of OCTA images generated in step S19.

The VISTA processor 232A executes the VISTA processing using the plurality OCTA image generated in step S19, and identifies the time variation of the motion contrast intensity in the cross-section. The VISTA processor 232A generates the VISTA image (for example, VISTA image VMA1 shown in FIG. 3, VISTA image VMA2 shown in FIG. 4) representing the distribution of the time variation of the identified motion contrast intensity as an analysis result. Further, the flow parameter analyzer 232B identifies the time change in the flow parameter(s) identified by the VISTA processor 232A, and generates the information representing the time change in flow parameter(s). The details of step S20 will be described below.

This terminates the operation of the ophthalmic apparatus 1 (END).

The processing in step S17 of FIG. 15 is performed on the same cross-section in the fundus Ef according to the flow shown in FIG. 16.

(S21: Perform OCT scan)

In step S17 of FIG. 15, first, the main controller 211 controls the OCT unit 100, etc. according to the scan condition(s) set in step S13, to perform a single line scan on the fundus Ef.

(S22: Has processing been repeated predetermined number of repetitions?)

Subsequently, the main controller determines whether or not the number of times the OCT scan in step S21 has been repeatedly performed on the same cross-section in the fundus Ef exceeds the predetermined number of repetitions in the scan condition set in step S13.

When it is determined that the number of times the OCT scan in step S21 has been repeatedly performed exceeds the predetermined number of repetitions of the scan condition (S22: Y), the processing in step S17 is terminated (END). On the other hand, when it is determined that the number of times the OCT scan in step S21 has been repeatedly performed does not exceed the predetermined number of repetitions of the scan condition (S22: N), the processing in step S17 proceeds to step S23.

(S23: Has interscan time elapsed?)

When it is determined in step S22 that the number of times the OCT scan in step S21 has been repeatedly performed does not exceed the predetermined number of repetitions of the scan condition (S22: N), the main controller 211 determines whether or not the interscan time has elapsed.

In other words, the main controller 211 determines whether or not the interscan time in the scan condition(s) set in step S13 has elapsed from the scan timing of the OCT scan performed in immediately preceding step S21.

When it is determined that the interscan time has elapsed (S23: Y), the processing in step S17 proceeds to step S21. On the other hand, when it is determined that the interscan time has not elapsed (S23: N), the processing in step S17 continues the processing in step S23.

As described above, the processing in step S17 of FIG. 15 is performed.

The processing in step S20 of FIG. 15 is performed according to the flow shown in FIG. 17.

(S31: Generate VISTA image)

The analyzer 232 generates the VISTA image according to the flow shown in FIG. 6, similar to the VISTA processor 1121.

Specifically, the VISTA processor 232A calculates the maximum value of the pixel values for each corresponding pixel in the respective OCTA images generated in step S19 as the statistical value calculator, as shown in FIG. 6. Further, the VISTA processor 232A adjusts the range of the luminance information as the range adjuster, and generates the luminance information by assigning the maximum values of the calculated pixel values to the luminance values in the range for each corresponding pixel as the luminance information generator.

Furthermore, the VISTA processor 232A identifies flow parameters for each corresponding pixel based on the OCTA images generated in step S19, as the flow parameter identifying unit, as shown in FIG. 6. Furthermore, the VISTA processor 232A adjusts the range of the hue information as the range adjuster, and generates the hue information by assigning the identified flow parameters to the hue values in the range for each corresponding pixel as the hue information generator.

The VISTA processor 232A generates the single VISTA image by assigning the generated luminance information and the generated hue information to the pixels for each pixel.

(S32: Perform time analysis of flow parameter)

Subsequently, the flow parameter analyzer 232B identifies the time change in a plurality of flow parameters identified in the designated pixel for each of the groups of OCTA images acquired at timings different from each other in step S19. The flow parameter analyzer 232B generates the information representing the time change in the flow parameters.

For example, in case of identifying the flow parameter at a pixel of interest, the flow parameter analyzer 232B performs weighted averaging on the pixels in the region designated by the user. Here, in the weighted averaging, the OCTA data of the corresponding pixel in the flow parameter image (VISTA image, image representing the time variation of the motion contrast intensity acquired by the VISTA processor 232A) is used. This makes it possible to analyze the time change in the variation of the motion contrast at the pixel of interest with high precision, without being affected by the surroundings of the pixel of interest, where the motion contrast intensity does not vary.

(S33: Display)

Next, the display controller 211A causes the VISTA image generated in step S31 and the information representing the time change in the flow parameter(s) generated is step S32 to be displayed on the display unit 240A.

As described above, the processing in step S20 of FIG. 15 is performed.

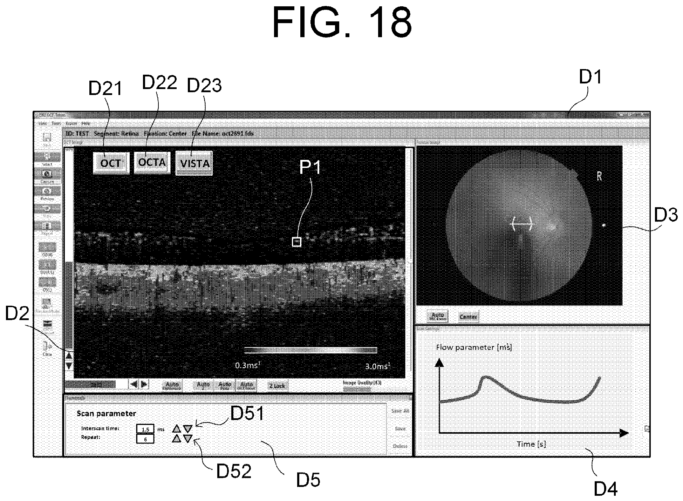

FIG. 18 schematically shows an example of a display for the display unit 240A in step S33.

The display controller 211A causes the image representing the analysis results obtained by the VISTA processor 232A to be displayed in an analysis result display area D1 on a display screen of the display unit 240A. The analysis result display area D1 includes a tomographic image display area D2, a live image display area D3, a flow parameter analysis result display area D4, and a scan parameter adjustment instruction area D5.

In the tomographic image display area D2, the VISTA image, etc. generated in step S31 is displayed. In the present embodiment, an OCT image display switching object D21, an OCTA image display switching object D22, and a VISTA image display switching object D23 are displayed in the tomographic image display area D2. When the user operates the operation unit 240B to designate a display switching object on the display screen, the display controller 211A causes an image of the type corresponding to the designated display switching object to be displayed in the tomographic image display area D2.

For example, when the user moves a cursor on the display screen by operating the operation unit 240B and performs a click operation, the main controller 211 determines whether or not a cursor position where the click operation has been performed is the display area of the display switching object. When it is determined that the cursor position is in the relevant display area, the main controller 211 identifies the type of the designated display switching object. The display controller 211A causes the image corresponding to the display switching object of the identified type to be displayed in the tomographic image display area D2.

Specifically, when the OCT image display switching object D21 is designated by the operation on the operation unit 240B, the display controller 211A causes the OCT image used for generating the VISTA image to be displayed in the tomographic image display area D2. The OCT image is, for example, any of the B-scan images IM1 to IM5 (B-scan images IM2 to IMG6). In some embodiments, the OCT image displayed in the tomographic image display area D2 is configured to be able to be further switched and be displayed from among the B-scan images IM1 to IM5 (B-scan images IM2 to IM6) by operating the operation unit 240B.

When the OCTA image display switching object D22 is designated by the operation on the operation unit 240B, the display controller 211A causes the OCTA image used for generating the VISTA image to be displayed in the tomographic image display area D2. The OCTA image is, for example, any of the OCTA images IMA11 to IMA14 (OCTA image IMA21 to IMA24). In some embodiments, the OCTA image displayed in the tomographic image display area D2 is configured to be able to be further switched and be displayed from among the OCTA images IMA11 to IMA14 (OCTA images IMA21 to IMA24) by operating the operation unit 240B.

When the VISTA image display switching object D23 is designated by the operation on the operation unit 240B, the display controller 211A causes the VISTA image to be displayed in the tomographic image display area D2. The VISTA image is, for example, the VISTA image VMA1 (VISTA image VMA2).

Here, the B-scan image, the OCTA image, and the VISTA image that are displayed in the tomographic image display area D2 are already aligned without performing position matching each other. Therefore, the morphology in the B-scan image and/or the motion contrast in the OCTA image which correspond to the region of interest in the VISTA image can be easily observed simply by operating to designate the display switching object.

In the live image display area D3, a projection image of the fundus Ef, an en-face image of the fundus Ef, or an observation image of the fundus Ef (or anterior segment Ea) that is acquired by the imaging optical system 30 is displayed. For example, an image indicating the scan position (scan range) can be superimposed and displayed on the live image displayed in the live image display area D3.

For example, the pixel value of each pixel in the projection image or the en-face image is the average or the maximum value of the pixel values in the depth direction. In this case, the data processor 230 performs a weighted averaging using the OCTA data of the corresponding pixel in the flow parameter image for each pixel to generate the projection image or the en-face image. Thereby, the vascular structures in a predetermined layer region can be depicted in detail, without being affected by regions other than the vascular region.

The pixel positions in the image displayed in the tomographic image display area D2 correspond to the pixel positions in the live image displayed in the live image display area D3. Therefore, by designating the pixel position in the image displayed in the tomographic image display area D2 using the operation unit 240B by the user, the display controller 211A can cause the pixel position of the corresponding pixel in the live image to be displayed in an identifiable manner. Further, by designating the pixel position in the live image displayed in the live image display area D3 using the operation unit 240B by the user, the display controller 211A can cause the pixel position of the corresponding pixel in the image displayed in the tomographic image display area D2 to be displayed in an identifiable manner.

In the flow parameter analysis result display area D4, the information representing the time change in the flow parameter is displayed. Examples of the information representing the time change in the flow parameter include a time change graph in which the vertical axis represents the flow parameter and the horizontal axis represents time.

In the scan parameter adjustment instruction area D5, a GUI for adjusting one or more scan parameters is displayed. The scan parameters include the interscan time, and the number of repetitions of the B-scan for the same cross-section. Here, the interscan time corresponds to the scan timing interval of the B-scan repeatedly performed for OCT.

For example, in the scan parameter adjustment instruction area D5, an interscan time adjustment object D51 and the number of repetitions adjustment object D52 are displayed. By designating the interscan time adjustment object D51 using the operation unit 240B by the user to change the interscan time, the interscan time is updated among the scan parameters. Similarly, by designating the number of repetitions adjustment object D52 using the operation unit 240B by the user to change the number of repetitions of the B-scan, the number of repetitions of the B-scan is updated among the scan parameters.

In the present embodiment, the VISTA processing is performed with a tomographic image-based approach on the fundus Ef of the eye E to be examined. Thereby, the analysis results of the VISTA can be obtained in real time. As a result, the scan parameter(s) required for more detailed analysis results can be easily adjusted, and new analysis results can be obtained in almost real time by reflecting such changes to the scan parameter(s) as described above.

For example, the user operates the operation unit 240B to designate the pixel position in the image displayed in the tomographic image display area D2 (pixel position P1 in FIG. 18), or the pixel position in the live image displayed in the live image display area D3. The main controller 211 controls the analyzer 232 in the data processor 230 to identify the time change in the flow parameter at the pixel position P1 as shown in FIG. 18, and to cause the image representing the time change in the flow parameter to be displayed on display unit 240A. The user repeats to adjust the scan parameters using the operation unit 240B as described above, to perform the OCT measurement to which the adjusted scan parameters are applied, and to perform the VISTA processing. This makes it easier to obtain analysis results that significantly show the time change in the flow parameter(s).

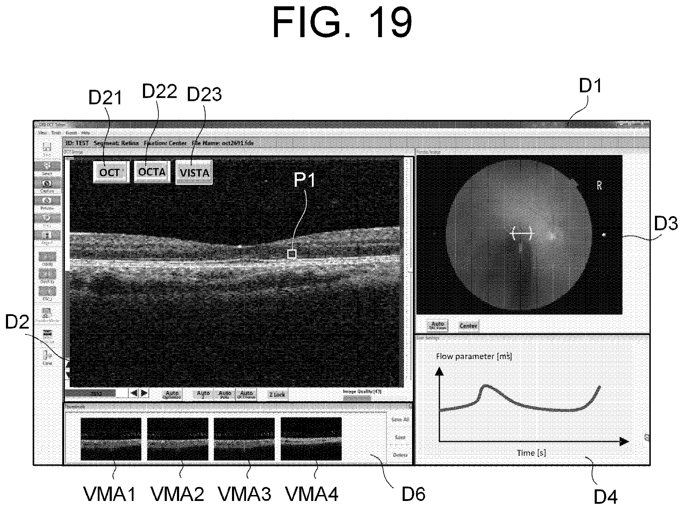

FIG. 19 schematically shows another example of the display for the display unit 240A in step S33. In FIG. 19, like reference numerals designate like parts as in FIG. 18. The same description may not be repeated.

The difference between the analysis result display area D1 shown in FIG. 19 and the analysis result display area D1 shown in FIG. 18 is that the VISTA image group display area D6 is provided instead of the scan parameter adjustment instruction area D5. It should be noted that one of the plurality of B-scan images used for generating the VISTA image is displayed in the tomographic image display area D2 in FIG. 19.

In the VISTA image group display area D6, the VISTA image group including a plurality of VISTA images (for example, VISTA images VMA1 to VMA4) sequentially formed from the OCTA image group acquired at timings different from each other is displayed simultaneously in chronological order. In some embodiments, the time variation of the motion contrast intensity is displayed as a moving image (for example, slow motion), by sequentially displaying the plurality of VISTA images at the same location in the VISTA image group display area D6 at a fixed time interval.

< Adjustment of scan parameter >

In FIG. 18, a case where the scan parameter is adjusted by the operation of the user has been described. However, the configuration according to the embodiments is not limited to this. For example, the scan parameter(s) may be configured to be automatically adjusted based on the VISTA analysis result(s).

In this case, the data processor 230 executes processing for adjusting the scan parameter(s) of OCT scan (B-scan) to obtain better analysis results of VISTA based on the VISTA analysis results obtained by the analyzer 232. In other words, the data processor 230 adjusts the scan parameter(s) of the OCT B-scan performed by controlling the OCT unit 100, etc. by the main controller 211, based on the time variation of the motion contrast intensity at each of one or more positions in the cross-section in the OCTA image. In some embodiments, the adjusted functions of the data processor 230 are performed by the controller 210 (main controller 211).

(First adjustment example of scan parameter)

In the embodiments, as the interscan time becomes longer, more vascular regions reach the saturated state of the decorrelation value described above corresponding to the time variation of the motion contrast intensity. On the other hand, the shorter the interscan time, the more vascular regions are in the state preceding the transition to the saturated state of the decorrelation value (see FIG. 8). Therefore, the data processor 230 adjusts the interscan time, as the scan parameter adjuster, so that the hue value is between the saturated state of the decorrelation value and a state of a lower detection limit of the decorrelation value, based on the hue value of the designated vascular region.

The main controller 211 causes the OCT measurement to be performed according to the scan parameters including the interscan time adjusted by the data processor 230, as shown in FIG. 16, and the VISTA processing to be performed based on the acquired measurement data. Then, in the acquired VISTA image, the adjustment of the interscan time, OCT re-measurement, and VISTA are repeated until the hue value in the designated vascular region described above is a desired hue value.

(Second adjustment example of scan parameter)

In the embodiments, as the interscan time becomes shorter, the amount of the change in the flow parameter(s) identified by the flow parameter analyzer 232B becomes smaller (see FIG. 18 or FIG. 19). Therefore, the data processor 230 adjusts the interscan time, as the scan parameter adjuster, so that the amount of the change in the flow parameter(s) is greater or equal to a predetermined threshold value.

The main controller 211 causes the OCT measurement to be performed according to the scan parameters including the interscan time adjusted by the data processor 230, as shown in FIG. 16, and the time analysis to be performed on the flow parameters after performing the VISTA processing based on the acquired measurement data. Then, the adjustment of the interscan time, OCT re-measurement, VISTA, and the time analysis of the flow parameter(s) are repeated until the obtained amount of the change in the time change of the flow parameter(s) is greater than or equal to the predetermined threshold value.

(Third adjustment example of scan parameter)

In the embodiment, the amount of the change of the time change in the flow parameter becomes larger in synchronization with the timing of the examinee's pulsation (see FIG. 18 or FIG. 19). Therefore, the data processor 230 adjusts the interscan time, as the scan parameter adjuster, so as to fit the pulsation cycle.

The main controller 211 causes the OCT measurement to be performed according to the scan parameters including the interscan time adjusted by the data processor 230, as shown in FIG. 16, and the time analysis to be performed on the flow parameters after performing the VISTA processing based on the acquired measurement data. Then, the adjustment of the interscan time, OCT re-measurement, VISTA, and the time analysis of the flow parameter(s) are repeated until the pulsation cycle of the examinee fits within the time change in the flow parameter(s).

(Fourth adjustment example of scan parameter)

The hue value assigned to each pixel in the VISTA image generated by VISTA processor 232A depends on the interscan time. Therefore, the data processor 230 adjusts the interscan time, as the scan parameter adjuster, so that the range of hue values in the entire VISTA image or in a predetermined region falls within a desired range.

The main controller 211 causes the OCT measurement to be performed according to the scan parameters including the interscan time adjusted by the data processor 230, as shown in FIG. 16, and the VISTA processing to be performed based on the acquired measurement data. Then, the adjustment of the interscan time, OCT re-measurement, and VISTA are repeated until the range of hue values in the entire VISTA image or in the predetermined region falls within the desired range.

(Fifth adjustment example of scan parameter)

The interscan time and the number of repetitions of the B-scan may be adjusted by specifically analyzing the change in the decorrelation value (or time variation of the motion contrast intensity) in the designated pixel or the designated region in the VISTA image. In the present adjustment example, after optimizing the interscan time at a predetermined designated pixel to obtain the optimum VISTA analysis results for a predetermined number of repetitions of the B-scan, the number of repetitions of the B-scan is assumed to be adjusted. Up to a predetermined number of times, as the number of repetitions of the B-scan increases, the accuracy of the VISTA analysis results can be improved. On the other hand, as the number of repetitions of the B-scan increases, the OCT measurement time, which depends on the OCT scan time specific to the ophthalmic apparatus 1, becomes longer, which increases the burden on the examinee. In the present adjustment example, the number of repetitions of the B-scan is assumed to be adjusted to be as few as possible within a predetermined maximum number of repetitions.

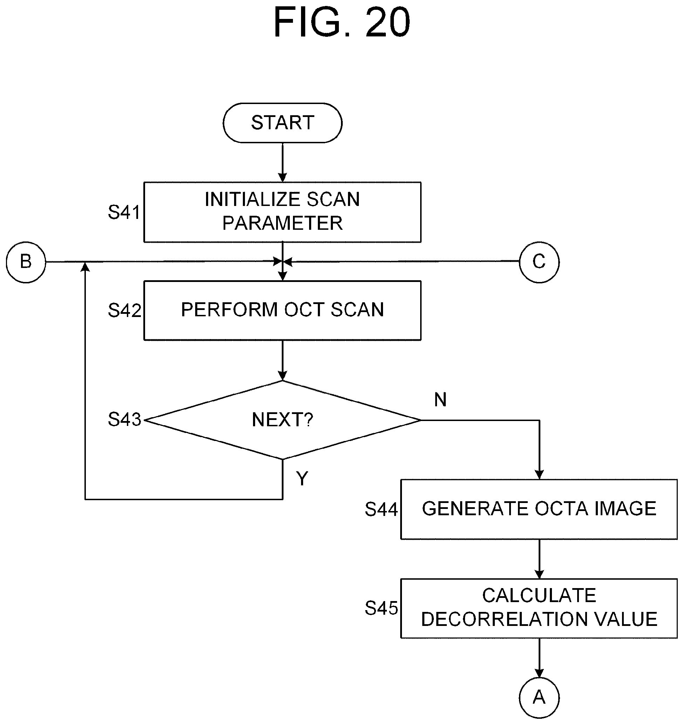

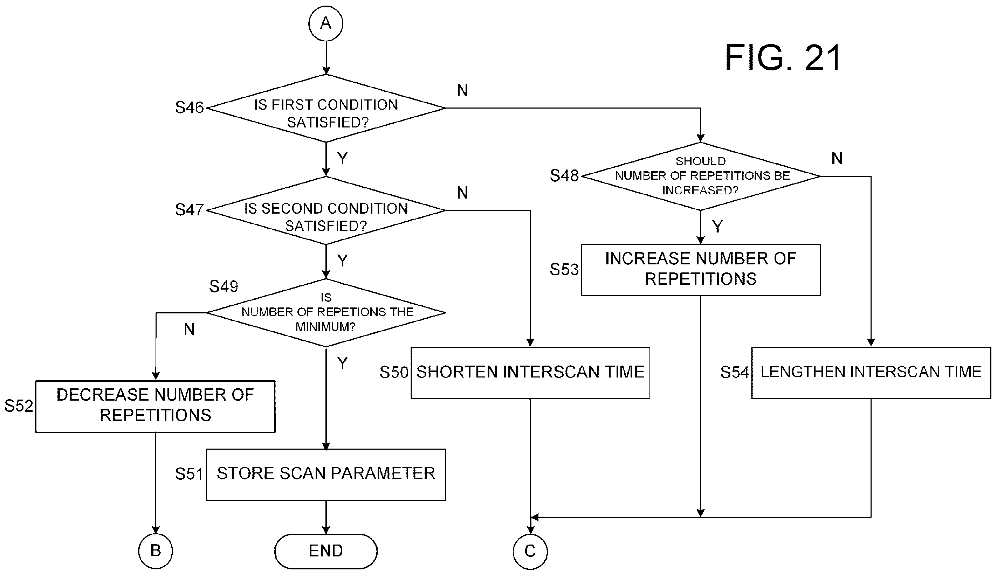

FIG. 20 and FIG. 21 show flowcharts of the fifth adjustment example of the scan parameter. The storage unit 212 stores computer program(s) for realizing the processing shown in FIG. 20 and FIG. 21. The main controller 211 operates according to the computer programs, and thereby the main controller 211 performs the processing shown in FIG. 20 and FIG. 21.

(S41: Initialize scan parameter)

First, the main controller 211 initializes the interscan time and the number of repetitions of the B-scan as the scan parameter.

(S42: Perform OCT scan)

Subsequently, the main controller 211 controls the OCT unit 100, etc. according to the scan parameters set at the time of executing step S42 to perform OCT scan. Thereby, the OCT data is acquired and the OCT image (B-scan image) is acquired.

(S43: Next?)

The main controller 211 determines whether or not the next OCT scan according to the number of repetitions in the scan parameters set at the time of executing step S43 should be performed.
When it is determined that the next OCT scan should be performed (step S43: Y), the processing according to the present adjustment example proceeds to step S42. When it is determined that the next OCT scan should not be performed (step S43: N), the processing according to the present adjustment example proceeds to step S44.

(S44: Generate OCTA image)

When it is determined in step S43 that the next OCT scan should not be performed (step S43: N), the main controller 211 controls the OCTA image generator 231 to generate a plurality of OCTA images at interscan time intervals different from each other, from the plurality of OCT images acquired by repeatedly executing step S42.

(S45: Calculate decorrelation value)

Subsequently, the main controller 211 controls the VISTA processor 232A to calculate the decorrelation value at the designated pixel for each of the plurality of OCTA images generated in step S44 (see FIG. 8).

(S46: Is first condition satisfied?)

Next, the main controller 211 determines whether or not the interscan time satisfies a predetermined first condition at the time of executing step S46. Here, the first condition is a condition that constrains the upper limit of the interscan time. In the present adjustment example, the first condition is a condition where "when sorting the decorrelation values in order of longer (shorter) interscan time, two or more consecutive decorrelation values including the longest interscan time are saturated". For example, it is possible to determine whether or not the two or more consecutive decorrelation values described above are saturated by determining whether or not the difference between the decorrelation values relative to the saturation level "D" for each of the two or more decorrelation values is within a predetermined threshold value.
When it is determined that the interscan time satisfies the first condition (S46: Y), the processing according to the present adjustment example proceeds to step S47. When it is determined that the interscan time does not satisfy the first condition (S46: N), the processing according to the present adjustment example proceeds to step S48.

(S47: Is second condition satisfied?)

When it is determined in step S46 that the interscan time satisfies the first condition (step S46: Y), the main controller 211 determines whether or not the interscan time satisfies a predetermined second condition at executing step at the time of executing step S47. Here, the second condition is a condition that constrains the lower limit of the interscan time. In the present adjustment example, the second condition is a condition where "the decorrelation value with the shortest interscan time is not saturated". For example, it is possible to determine whether or not the decorrelation value with the shortest interscan time is not saturated by determining whether or not the decorrelation value with the shortest interscan time is smaller than the decorrelation value with the next shortest interscan time.
When it is determined that the interscan time satisfies the second condition (S47: Y), the processing according to the present adjustment example proceeds to step S49. When it is determined that the interscan time does not satisfy the second condition (S47: N), the processing according to the present adjustment example proceeds to step S50.

(S48: Should number of repetitions be increased?)

When it is determined in step S46 that the interscan time does not satisfy the first condition (S46: N), the main controller 211 determines whether or not the number of repetitions of the B-scan should be increased. For example, the main controller 211 determines that the number of repetitions of the B-scan should be increased when the number of repetitions of the B-scan is less than or equal to a predetermined maximum number of repetitions of the B-scan at the time of executing step S48. The main controller 211 determines that the number of repetitions of the B-scan should not be increased when the number of repetitions of the B-scan is the predetermined maximum number of repetitions.
When it is determined in step S48 that the number of repetitions of the B-scan should be performed (S48: Y), the processing according to the present adjustment example proceeds to step S53. When it is determined in step S48 that the number of repetitions of the B-scan should not be performed (S48: N), the processing according to the present adjustment example proceeds to step S54.

(S49: Is number of repetitions the minimum?)

When it is determined in step S47 that the interscan time satisfies the second condition (S47: Y), the main controller 211 determines whether or not the number of repetitions of the B-scan is the minimum at the time of executing step S49. For example, the minimum number of repetitions of the B-scan is determined in advance in order to obtain analysis results with a predetermined level of accuracy or higher. The main controller 211 determines that the number of repetitions at present is the minimum when the number of repetitions of the B-scan is a predetermined minimum number of repetitions at the time of executing step S49. The main controller 211 determines that the number of repetitions of the B-scan is not the minimum when the number of repetitions of the B-scan is greater than the predetermined minimum of repetitions.
When it is determined that the number of repetitions of the B-scan is the minimum at the time of executing step S49 (S49: Y), the processing according to the present adjustment example proceeds to step S51. When it is determined that the number of repetitions of the B-scan is not the minimum at the time of executing step S49 (S49: N), the processing according to the present adjustment example proceeds to step S52.

(S50: Shorten interscan time)

When it is determined in step S47 that the interscan time does not satisfy the second condition (S47: N), the main controller 211 shortens the interscan time by a predetermined time. Subsequent to step S50, the processing according to the present adjustment example proceeds to step S42.

(S51: Store scan parameter)

When it is determined in step S49 that the number of repetitions of the B-scan is the minimum (S49: Y), the main controller 211 sets, as new scan parameters, the interscan time and the number of repetitions of the B-scan at the time of executing step S51 for the scan condition(s). The updated the scan condition(s) is/are stored in the storage unit 212.
After step S51, the processing according to the present adjustment example is terminated (END).

(S52: Decrease number of repetitions)

When it is determined in step S49 that the number of repetitions of the B-scan is not the minimum (S49: N), the main controller 211 decreases the number of repetitions by one. Subsequent to step S52, the processing according to the present adjustment example proceeds to step S42.

(S53: Increase number of repetitions)

When it is determined in step S48 that the number of repetitions of the B-scan should be increased (S48: Y), the main controller 211 increases the number of repetitions of the B-scan by one. Subsequent to step S53, the processing according to the present adjustment example proceeds to step S42.

(S54: Lengthen interscan time)

When it is determined in step S48 that the number of repetitions of the B-scan should not be increased (S48: N), the main controller 211 lengthens the number of repetitions of the B-scan by a predetermined time. Subsequent to step S54, the processing according to the present adjustment example proceeds to step S42.

In the fifth adjustment example, an example where the scan parameter is adjusted using the decorrelation value at the designated pixel in the VISTA image has been described. However, the fifth adjustment example is not limited to this. For example, for each of a plurality of pixels in a designated region in the VISTA image, the optimum scan parameters for each pixel may be identified as shown in FIG. 20 and FIG. 21, and the statistical value, such as mode and median, of the identified scan parameters may be adjusted as new scan parameters. In other words, the scan parameter(s) of the B-scan may be adjusted based on the time variation of the motion contrast intensity at each of one or more positions in the cross-section of the fundus Ef. The designated region may be the entire VISTA image or a region of interest in the VISTA image. The plurality of pixels in the designated region may be all pixels in the designated region.

As described above, according to the embodiments, the VISTA processing is performed with the B-scan image-based approach. Thereby, the analysis results of the VISTA can be generated in real time. As a result, the need to acquire volume data is eliminated, and the scan parameter(s) can be easily adjusted. And useful analysis results of the VISTA can be easily obtained.

The data processor 230 (or the data processor 230 and controller 210) is an example of the "adjuster" according to the embodiments.

[Actions]

The analysis processing apparatus, the optical coherence tomography apparatus, the analysis processing method, and the program according to the embodiments will be described.

The first aspect according to the embodiments is an analysis processing apparatus (1100, analysis processor 1100a, arithmetic control unit 200 (controller 210, image forming unit 220, and data processor 230)) includes an acquisition unit (1110), an analyzer (1120, 232), and a display controller (1130, 211A). The acquisition unit is configured to acquire two or more pieces of motion contrast data (OCTA images) of a cross-section at interscan time intervals different from each other. Here, the two or more pieces of motion contrast data are acquired by repeatedly performing B-scans three or more times on the same cross-section in an object to be measured (fundus Ef). The analyzer is configured to perform analysis processing (VISTA) for obtaining a time variation of a motion contrast intensity in the cross-section based on the two or more pieces of motion contrast data. The display controller is configured to cause an image (VISTA image) representing the time variation obtained by performing the analysis processing to be displayed on a display means (display apparatus 3, display unit 240A).

According to such an aspect, the analysis processing for obtaining the time variation of the motion contrast intensity in the cross-section in the object to be measured with the B-scan image-based approach. Thereby, the measurement time and the time required for analysis can be significantly reduced, and the amount of data required for analysis can also be significantly decreased. As a result, the analysis results can be generated in real time. In addition, since the results of the analysis processing can be generated in real time, the scan parameters including the interscan time and the number of repetitions of the B-scan can be easily optimized, which also can improve the accuracy of the analysis results.

In the second aspect according to the embodiments, in the first aspect, the acquisition unit is configured to acquire two or more contrast data groups (OCTA image groups) acquired at timings different from each other. Here, each of the two or more contrast data groups includes the two or more pieces of motion contrast data. The analyzer is configured to sequentially perform the analysis processing on the each of the two or more contrast data groups. The display controller is configured to cause two or more images, each of the two or more images representing the time variation obtained by performing the analysis processing, to be simultaneously displayed on a screen of the display means, or to cause the two or more images, each of the two or more images representing the time variation, at a same position to be sequentially displayed on the display means.

According to such an aspect, the time change in the time variation of the motion contrast intensity can be easily grasped visually.

In the third aspect according to the embodiments, in the first aspect or the second aspect, the analyzer is configured to generate the image representing the time variation described above in real time.

According to such an aspect, the position where the motion contrast intensity temporally varies can be grasped in real time.

In the fourth aspect according to the embodiments, in the first aspect or the second aspect, the analyzer is configured to generate the image representing the time variation based on a saturation time of the motion contrast intensity at each of positions in the cross-section.

According to such an aspect, for each site of the object to be measured, a site with a relatively fast change speed in the motion contrast and a site with a relatively slow change speed in the motion contrast can be easily grasped.

In the fifth aspect according to the embodiments, the analyzer is configured to generate the image representing a time change in the time variation (time change in the flow parameter) at a designated position in the cross-section.

According to such an aspect, the relative change speed of the motion contrast at the designated position can be easily grasped.

In the sixth aspect according to the embodiments, in the fifth aspect, the display controller is configured to cause information representing the time change in the time variation to be displayed on the display means.

According to such an aspect, the relative change speed of the motion contrast at the designated position can be easily grasped.

The seventh aspect according to the embodiments, in the first aspect or the second aspect, further includes an adjuster (data processor 230, data processor 230 and controller 210) configured to adjust one or more parameters of the B-scan based on the time variation of the motion contrast intensity at each of one or more positions in the cross-section.

According to such an aspect, the scan parameter that can satisfactorily identify the time variation of the motion contrast intensity in the object to be measured can be easily adjusted.

In the eighth aspect according to the embodiments, in the seventh aspect, the one or more scan parameters include an interscan time, and number of repetitions of the B-scan for a same cross-section.

According to such an aspect, by adjusting the interscan time and the number of repetitions of the B-scan, it becomes easy to satisfactorily identify the time variation of the motion contrast intensity in the object to be measured.

The ninth aspect according to the embodiments is an optical coherence tomography apparatus (1400) including an optical system (OCT optical system 1210, optical system from objective lens 22 to OCT unit 100 shown in FIG. 10), and the analysis processing apparatus of the first aspect or the second aspect. The optical system is configured to perform optical coherence tomography on the object to be measured. The analysis processing apparatus is configured to cause an image representing the time variation to be displayed on the display means (display apparatus 3, display unit 240A) based on the two or more pieces of motion contrast data acquired by repeatedly performing three or more B-scans on the same cross-section in the object to be measured using the optical system.

According to such an aspect, the analysis processing for obtaining the time variation of the motion contrast intensity in the cross-section in the object to be measured with the B-scan image-based approach. Thereby, the measurement time and the time required for analysis can be significantly reduced, and the amount of data required for analysis can also be significantly decreased. As a result, the analysis results can be generated in real time. In addition, since the results of the analysis processing can be generated in real time, the scan parameters including the interscan time and the number of repetitions of the B-scan can be easily optimized, which also can improve the accuracy of the analysis results.

The tenth aspect according to the embodiments is an analysis processing method including an acquisition step, an analyzing step, and a display control step. The acquisition step is performed to acquire two or more pieces of motion contrast data (OCTA images) of a cross-section at interscan time intervals different from each other. Here, the two or more pieces of motion contrast data are acquired by repeatedly performing B-scans three or more times on the same cross-section in an object to be measured (fundus Ef). The analyzing step is performed to perform analysis processing for obtaining a time variation of a motion contrast intensity in the cross-section based on the two or more pieces of motion contrast data. The display control step is performed to cause an image representing the time variation obtained by performing the analysis processing to be displayed on a display means (display apparatus 3, display unit 240A).

According to such an aspect, the analysis processing for obtaining the time variation of the motion contrast intensity in the cross-section in the object to be measured with the B-scan image-based approach. Thereby, the measurement time and the time required for analysis can be significantly reduced, and the amount of data required for analysis can also be significantly decreased. As a result, the analysis results can be generated in real time. In addition, since the results of the analysis processing can be generated in real time, the scan parameters including the interscan time and the number of repetitions of the B-scan can be easily optimized, which also can improve the accuracy of the analysis results.

In the eleventh aspect according to the embodiments, in the tenth aspect, the acquisition step is performed to acquire two or more contrast data groups acquired at timings different from each other. Here, each of the two or more contrast data group includes the two or more pieces of motion contrast data. The analyzing step is performed to sequentially perform the analysis processing on the each of the two or more contrast data groups. The display control step is performed to cause two or more images, each of the two or more images representing the time variation obtained by performing the analysis processing, to be simultaneously displayed on a screen of the display means, or to cause the two or more images, each of the two or more images representing the time variation, at a same position to be sequentially displayed on the display means.

According to such an aspect, the time change in the time variation of the motion contrast intensity can be easily grasped visually.

In the twelfth aspect according to the embodiments, in the tenth aspect or the eleventh aspect, the analyzing step is performed to generate the image representing the time variation in real time.

According to such an aspect, the position where the motion contrast intensity temporally varies can be grasped in real time.

In the thirteenth aspect according to the embodiments, in the tenth aspect or the eleventh aspect, the analyzing step is performed to generate the image representing the time variation based on a saturation time of the motion contrast intensity at each of positions in the cross-section.

According to such an aspect, for each site of the object to be measured, a site with a relatively fast change speed in the motion contrast and a site with a relatively slow change speed in the motion contrast can be easily grasped.

In the fourteenth aspect according to the embodiments, in the tenth aspect or the eleventh aspect, the analyzing step is performed to generate information representing a time change in the time variation at a designated position in the cross-section.

According to such an aspect, the relative change speed of the motion contrast at the designated position can be easily grasped.

In the fifteenth aspect according to the embodiments, in the fourteenth aspect, the display control step is performed to cause information representing the time change in the time variation to be displayed on the display means.

According to such an aspect, the relative change speed of the motion contrast at the designated position can be easily grasped.

The sixteenth aspect according to the embodiments, in the tenth aspect or the eleventh aspect, further includes an adjusting step of adjusting one or more scan parameters of the B-scan based on the time variation of the motion contrast intensity at each of one or more positions in the cross-section.

According to such an aspect, the scan parameter that can satisfactorily identify the time variation of the motion contrast intensity in the object to be measured can be easily adjusted.

In the seventeenth aspect according to the embodiments, in the sixteenth aspect, the one or more scan parameters include an interscan time, and number of repetitions of the B-scan for a same cross-section.

According to such an aspect, by adjusting the interscan time and the number of repetitions of the B-scan, it becomes easy to satisfactorily identify the time variation of the motion contrast intensity in the object to be measured.

The eighteenth aspect according to the embodiments is a program of causing a computer to execute each step of the analysis processing method of the tenth aspect or the eleventh aspect.

According to such an aspect, the analysis processing for obtaining the time variation of the motion contrast intensity in the cross-section in the object to be measured with the B-scan image-based approach. Thereby, the measurement time and the time required for analysis can be significantly reduced, and the amount of data required for analysis can also be significantly decreased. As a result, the analysis results can be generated in real time. In addition, since the results of the analysis processing can be generated in real time, the scan parameters including the interscan time and the number of repetitions of the B-scan can be easily optimized, which also can improve the accuracy of the analysis results.

The embodiments described above are merely examples. One who intends to implement the present invention may arbitrarily modify (omission, replacement, addition, etc.) within the scope of the invention.

In some embodiments, a program that causes a computer to execute an analysis processing method is stored in the storage unit 212. Such a program may be stored on any non-transitory recording medium that can be read by a computer. The recording medium may be an electronic medium using magnetism, light, magneto-optical, semiconductor, or the like. Typically, the recording medium is a magnetic tape, a magnetic disk, an optical disk, a magneto-optical disk, a flash memory, a solid state drive, or the like.

[EXPLANATION OF SYMBOLS]

1 Ophthalmic apparatus
210 Controller
230 Data processor
231 OCTA image generator
232, 1120 Analyzer
232A, 1121 VISTA processor
232B Flow parameter analyzer
1000, 1000a Analysis system
1100 Analysis processing apparatus
1100a Analysis processor

1110 Acquisition unit
1122 Flow parameter analyzer
1200, 1400 OCT apparatus
1210 OCT optical system
1300 Display apparatus
IM1 to IM7 B-scan image (OCT image)
IMA11 to IMA14, IMA21 to IMA24 OCTA image
VMA1, VMA2 VISTA image

**Claims**

1. An analysis processing apparatus, comprising:

   an acquisition unit configured to acquire two or more pieces of motion contrast data of a cross-section at interscan time intervals different from each other, the two or more pieces of motion contrast data being acquired by repeatedly performing B-scans three or more times on the same cross-section in an object to be measured;
   an analyzer configured to perform analysis processing for obtaining a time variation of a motion contrast intensity in the cross-section based on the two or more pieces of motion contrast data; and
   a display controller configured to cause an image representing the time variation obtained by performing the analysis processing to be displayed on a display means.

2. The analysis processing apparatus of claim 1, wherein

   the acquisition unit is configured to acquire two or more contrast data groups acquired at timings different from each other, each of the two or more contrast data groups including the two or more pieces of motion contrast data, the analyzer is configured to sequentially perform the analysis processing on the each of the two or more contrast data groups, and
   the display controller is configured to cause two or more images, each of the two or more images representing the time variation obtained by performing the analysis processing, to be simultaneously displayed on a screen of the display means, or to cause the two or more images, each of the two or more images representing the time variation, at a same position to be sequentially displayed on the display means.

3. The analysis processing apparatus of claim 1 or 2, wherein
   the analyzer is configured to generate the image representing the time variation in real time.

4. The analysis processing apparatus of claim 1 or 2, wherein
   the analyzer is configured to generate the image representing the time variation based on a saturation time of the motion contrast intensity at each of positions in the cross-section.

5. The analysis processing apparatus of claim 1 or 2, wherein
   the analyzer is configured to generate the image representing a time change in the time variation at a designated position in the cross-section.

6. The analysis processing apparatus of claim 5, wherein
   the display controller is configured to cause information representing the time change in the time variation to be displayed on the display means.

7. The analysis processing apparatus of claim 1 or 2, further comprising
   an adjuster configured to adjust one or more parameters of the B-scan based on the time variation of the motion contrast intensity at each of one or more positions in the cross-section.

8. The analysis processing apparatus of claim 7, wherein
   the one or more scan parameters include an interscan time, and number of repetitions of the B-scan for a same cross-section.

9. An optical coherence tomography apparatus, comprising:

an optical system configured to perform optical coherence tomography on the object to be measured; and the analysis processing apparatus of claim 1 or 2, the analysis processing apparatus being configured to cause an image representing the time variation to be displayed on the display means based on the two or more pieces of motion contrast data acquired by repeatedly performing three or more B-scans on the same cross-section in the object to be measured using the optical system.

10. An analysis processing method, comprising:

an acquisition step of acquiring two or more pieces of motion contrast data of a cross-section at interscan time intervals different from each other, the two or more pieces of motion contrast data being acquired by repeatedly performing B-scans three or more times on the same cross-section in an object to be measured; an analyzing step of performing analysis processing for obtaining a time variation of a motion contrast intensity in the cross-section based on the two or more pieces of motion contrast data; and a display control step of causing an image representing the time variation obtained by performing the analysis processing to be displayed on a display means.

11. The analysis processing method of claim 10, wherein

the acquisition step is performed to acquire two or more contrast data groups acquired at timings different from each other, each of the two or more contrast data group including the two or more pieces of motion contrast data, the analyzing step is performed to sequentially perform the analysis processing on the each of the two or more contrast data groups, and the display control step is performed to cause two or more images, each of the two or more images representing the time variation obtained by performing the analysis processing, to be simultaneously displayed on a screen of the display means, or to cause the two or more images, each of the two or more images representing the time variation, at a same position to be sequentially displayed on the display means.

12. The analysis processing method of claim 10 or 11, wherein the analyzing step is performed to generate the image representing the time variation in real time.

13. The analysis processing method of claim 10 or 11, wherein the analyzing step is performed to generate the image representing the time variation based on a saturation time of the motion contrast intensity at each of positions in the cross-section.

14. The analysis processing method of claim 10 or 11, wherein the analyzing step is performed to generate information representing a time change in the time variation at a designated position in the cross-section.

15. The analysis processing method of claim 14, wherein the display control step is performed to cause information representing the time change in the time variation to be displayed on the display means.

16. The analysis processing method of claim 10 or 11, further comprising an adjusting step of adjusting one or more scan parameters of the B-scan based on the time variation of the motion contrast intensity at each of one or more positions in the cross-section.

17. The analysis processing method of claim 16, wherein the one or more scan parameters include an interscan time, and number of repetitions of the B-scan for a same cross-section.

18. A program of causing a computer to execute each step of the analysis processing method of claim 10 or 11.

# FIG. 1

1000

1200

OCT
APPARATUS

1100

ANALYSIS
PROCESSING
APPARATUS

1300

DISPLAY
APPARATUS

# FIG. 2

1100

ANALYSIS PROCESSING APPARATUS

| ACQUISITION UNIT | — 1110 |

1120

ANALYZER

| VISTA PROCESSOR | — 1121 |

| FLOW PARAMETER ANALYZER | — 1122 |

| DISPLAY CONTROLLER | — 1130 |

# FIG. 3

EP 4 736 739 A1

# FIG. 4

EP 4 736 739 A1

# FIG. 5

1121

```
VISTA PROCESSOR

  STATISTICAL VALUE
  CACLULATOR                          1121A

  RANGE ADJUSTER                      1121B

  LUMINANCE INFORMATION
  GENERATOR                           1121C

  FLOW PARAMETER IDENTIFYING
  UNIT                                1121D

  RANGE ADJUSTER                      1121E

  HUE INFORMATION GENERATOR           1121F
```

# FIG. 6

IMA1　　　　IMA2　　　　IMA3　　　　IMA4

p1-1　　　　p1-2　　　　p1-3　　　　p1-4

| CALCULATION OF STATISTICAL VALUE | SQ1 |
|---|---|

| IDENTIFICATION OF FLOW PARAMETER | SQ11 |
|---|---|

| ADJUSTMENT OF RANGE | SQ2 |
|---|---|

| ADJUSTMENT OF RANGE | SQ12 |
|---|---|

| GENERATION OF LUMINANCE INFORMATION | SQ3 |
|---|---|

| GENERATION OF HUE INFORMATION | SQ13 |
|---|---|

| VISTA IMAGE | SQ20 |
|---|---|

# FIG. 7

IMA

# FIG. 8

# FIG. 9

1000a

1400

OCT APPARATUS

OCT OPTICAL
SYSTEM — 1210

ANALYSIS
PROCESSOR — 1100a

1300

DISPLAY
APPARATUS

FIG. 10

EP 4 736 739 A1

# FIG. 11

# FIG. 12

EP 4 736 739 A1

# FIG. 13

230

DATA PROCESSOR

| OCTA IMAGE GENERATOR | 231 |

| ANALYZER | 232 |

# FIG. 14

232

ANALYZER

| VISTA PROCESSOR | 232A |

| FLOW PARAMETER ANALYZER | 232B |

# FIG. 15

START

S11 PERFORM ALIGNMENT

S12 START TRACKING

S13 SET SCAN CONDITION

S14 ACQUIRE TOMOGRAPHIC IMAGE FOR ADJUSTMENT

S15 ADJUST REFERENCE POSITION IN DEPTH DIRECTION

S16 PERFORM FOCUS ADJUSTMENT / PERFORM POLARIZATION ADJUSTMENT

S17 PERFORM OCT MEASUREMENT

S18 GENERATE OCT IMAGE

S19 GENERATE OCTA IMAGE

S20 PERFORM VISTA PROCESSING

END

# FIG. 16

```
            ┌─────────────┐
            │    START    │
            └──────┬──────┘
                   │
        ┌──────────▼──────────┐
  S21   │  PERFORM OCT SCAN   │
        └──────────┬──────────┘
                   │
                 ╱─▼─╲
               ╱  HAS  ╲
  S22      ╱ PROCESSING BEEN REPEATED ╲   N
          ◁ PREDETERMINED NUMBER OF ▷──────────┐
           ╲   REPETITIONS?       ╱            │
             ╲───┬───╱                         │
                 │ Y                         ╱─▼─╲
                 │                    S23  ╱       ╲   N
          ┌──────▼──────┐               ◁ HAS INTERSCAN TIME ▷──┐
          │     END     │                ╲   ELAPSED?        ╱  │
          └─────────────┘                  ╲─────┬───╱          │
                                                 │ Y
```

# FIG. 17

```
        ┌─────────────┐
        │    START    │
        └──────┬──────┘
               │
               ▼
S31 ┌──────────────────────────┐
    │   GENERATE VISTA IMGE     │
    └──────────┬───────────────┘
               │
               ▼
S32 ┌──────────────────────────┐
    │ PERFORM TIME ANALYSIS OF  │
    │     FLOW PARAMETER        │
    └──────────┬───────────────┘
               │
               ▼
S33 ┌──────────────────────────┐
    │        DISPLAY            │
    └──────────┬───────────────┘
               │
               ▼
        ┌─────────────┐
        │     END     │
        └─────────────┘
```

# FIG. 18

# FIG. 19

# FIG. 20

```
                    START

            S41   INITIALIZE SCAN
                  PARAMETER

 B                                              C

            S42   PERFORM OCT SCAN


                                        N
            S43        NEXT?

                         Y
                                  S44   GENERATE OCTA IMAGE


                                  S45   CALCULATE
                                        DECORRELATION VALUE

                                                A
```

# FIG. 21

EP 4 736 739 A1

## INTERNATIONAL SEARCH REPORT

| International application No. |
| --- |
| **PCT/JP2024/021492** |

| A. | CLASSIFICATION OF SUBJECT MATTER |
| --- | --- |

*A61B 3/10*(2006.01)i; *A61B 3/12*(2006.01)i
FI:   A61B3/10 100; A61B3/12 300

According to International Patent Classification (IPC) or to both national classification and IPC

| B. | FIELDS SEARCHED |
| --- | --- |

Minimum documentation searched (classification system followed by classification symbols)

A61B3/10; A61B3/12

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | JP 2023-77981 A (CANON KABUSHIKI KAISHA) 06 June 2023 (2023-06-06) entire text, all drawings | 1-18 |
| A | JP 2022-155690 A (CANON KABUSHIKI KAISHA) 14 October 2022 (2022-10-14) entire text, all drawings | 1-18 |
| A | JP 2022-11912 A (CANON KABUSHIKI KAISHA) 17 January 2022 (2022-01-17) entire text, all drawings | 1-18 |
| A | US 2021/0145277 A1 (OREGON HEALTH & SCIENCE UNIVERSITY) 20 May 2021 (2021-05-20) entire text, all drawings | 1-18 |
| A | US 2018/0315194 A1 (MASSACHUSETTS INSTITUTE OF TECHNOLOGY) 01 November 2018 (2018-11-01) entire text, all drawings | 1-18 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **02 August 2024** | **13 August 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

| International application No. |
|---|
| **PCT/JP2024/021492** |

### C. DOCUMENTS CONSIDERED TO BE RELEVANT

| Category* | Citation of document, with indication, where appropriate, of the relevant passages | Relevant to claim No. |
|---|---|---|
| A | WO 2022/169722 A1 (OREGON HEALTH & SCIENCE UNIVERSITY) 11 August 2022 (2022-08-11) <br> entire text, all drawings | 1-18 |

Form PCT/ISA/210 (second sheet) (July 2022)

**INTERNATIONAL SEARCH REPORT**
Information on patent family members

International application No.

**PCT/JP2024/021492**

| Patent document cited in search report | | | Publication date (day/month/year) | Patent family member(s) | Publication date (day/month/year) |
|---|---|---|---|---|---|
| JP | 2023-77981 | A | 06 June 2023 | (Family: none) | |
| JP | 2022-155690 | A | 14 October 2022 | (Family: none) | |
| JP | 2022-11912 | A | 17 January 2022 | (Family: none) | |
| US | 2021/0145277 | A1 | 20 May 2021 | (Family: none) | |
| US | 2018/0315194 | A1 | 01 November 2018 | (Family: none) | |
| WO | 2022/169722 | A1 | 11 August 2022 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180315194
- US 20200064118

- JP 2013248376 A

**Non-patent literature cited in the description**

- **STEFAN B. PLONER et al.** Toward quantitative OCT angiography: visualizing blood flow speeds in ocular pathology using variable interscan time analysis (VISTA). *Retina. Auther manuscript; available in PMC*, 01 December 2017